(19) Europäisches Patentamt / European Patent Office / Office européen des brevets

(11) **EP 3 142 549 B1**

(12) **FASCICULE DE BREVET EUROPEEN**

(45) Date de publication et mention de la délivrance du brevet:
**06.09.2023 Bulletin 2023/36**

(21) Numéro de dépôt: **15732373.4**

(22) Date de dépôt: **15.05.2015**

(51) Classification Internationale des Brevets (IPC):
*A61B 5/029* (2006.01)      *A61B 5/021* (2006.01)
*A61B 8/06* (2006.01)      *A61B 5/11* (2006.01)
*A61B 5/0215* (2006.01)

(52) Classification Coopérative des Brevets (CPC):
**A61B 5/021; A61B 5/029; A61B 5/1107;**
A61B 5/02158

(86) Numéro de dépôt international:
**PCT/IB2015/053608**

(87) Numéro de publication internationale:
**WO 2015/173785 (19.11.2015 Gazette 2015/46)**

(54) **DISPOSITIF POUR EVALUER EN CONTINUE LE COUPLAGE VENTRICULO-AORTIQUE DE PATIENTS A RISQUE, PAR ANALYSE DE BOUCLES PRESSION-FLUX**

VORRICHTUNG ZUR KONTINUIERLICHEN BEURTEILUNG DER VENTRIKEL-AORTA-KOPPLUNG VON GEFÄHRDETEN PATIENTEN MITTELS ANALYSE VON DRUCKSFLUSSSCHLEIFEN

DEVICE FOR THE CONTINUOUS EVALUATION OF THE VENTRICULO-AORTIC COUPLING OF AT-RISK PATIENTS BY ANALYSIS OF PRESSURE-FLOW LOOPS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.05.2014 FR 1454338**

(43) Date de publication de la demande:
**22.03.2017 Bulletin 2017/12**

(73) Titulaires:
• **ASSISTANCE PUBLIQUE HOPITAUX DE PARIS**
**75012 Paris 12 (FR)**
• **Université Paris Cité**
**75006 Paris (FR)**

(72) Inventeurs:
• **VALLEE, Fabrice**
**F-93260 Les Lilas (FR)**
• **MEBAZZA, Alexandre**
**F-92120 Montrouge (FR)**
• **GAYAT, Etienne**
**F-75019 Paris (FR)**
• **LE GALL, Arthur**
**F-75013 Paris (FR)**
• **JOACHIM, Jona**
**92300 Levallois-Perret (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**WO-A2-03/037181      US-A1- 2007 161 914**

• **THIELE ROBERT H ET AL: "Real-Time Doppler-Based Arterial Vascular Impedance and Peripheral Pressure-Flow Loops: A Pilot Study", JOURNAL OF CARDIOTHORACIC AND VASCULAR ANESTHESIA, SAUNDERS, PHILADELPHIA, PA, US, vol. 28, no. 1, 25 septembre 2013 (2013-09-25), pages 36-41, XP028809187, ISSN: 1053-0770, DOI: 10.1053/J.JVCA.2013.04.021**
• **T. NOZAWA ET AL: "Efficiency of energy transfer from pressure-volume area to external mechanical work increases with contractile state and decreases with afterload in the left ventricle of the anesthetized closed-chest dog", CIRCULATION, vol. 77, no. 5, 1 mai 1988 (1988-05-01), pages 1116-1124, XP055163324, ISSN: 0009-7322, DOI: 10.1161/01.CIR.77.5.1116**

**Description**

La présente invention concerne le domaine de la réanimation cardio-vasculaire. Plus précisément, elle présente une nouvelle méthode de suivi du couplage ventriculo-aortique de patients à risque, qui peut être mise en oeuvre avec les appareils de monitorage couramment utilisés au bloc opératoire, en réanimation et dans les services d'urgences.

Le choix de la stratégie optimale pour la réanimation cardio-vasculaire (expansion volémique ou administration de vasoconstricteurs, choix du vasoconstricteur, choix du volume ou de la dose de médicament, *etc.*) des patients à risque ou des patients instables est un enjeu majeur mais reste compliqué. En effet, il n'existe pas, aujourd'hui, de réel consensus sur le choix des différents paramètres à surveiller, sur les moyens de les mesurer ni sur les valeurs "cibles" à obtenir. Il est certes recommandé de monitorer la pression artérielle moyenne en continu (PAM) chez ce type de patients et d'obtenir une valeur minimale autour de 65 mm Hg, mais le débat reste ouvert quant au seuil réel de PAM à obtenir (65 ou 85mm Hg : [1]). De plus, une quantité croissante de littérature suggère que guider la thérapeutique pour optimiser le débit cardiaque (DC) dans la période péri-opératoire des patients à risque ou des patients instables peut améliorer le pronostic de ces patients [2]. De tous les appareils disponibles, le Doppler trans-oesophagien (DTO), qui mesure de débit sanguin dans l'aorte thoracique, semble être le moniteur du débit cardiaque associé avec la reproductibilité la plus convaincante, tout en restant peu invasif. Pour les patients à risque cardiovasculaire, un monitorage du débit cardiaque, via le DTO, associé à une mesure invasive de la PAM par un cathéter artériel, est donc une attitude actuellement recommandée.

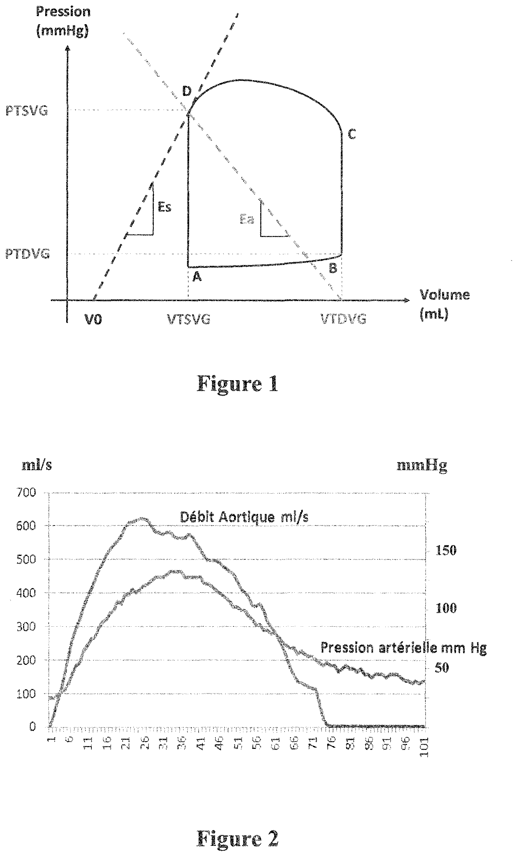

Le couplage ventriculo-aortique est un concept physiologique permettant d'intégrer les interactions qui existent entre le coeur et les vaisseaux lors d'un cycle cardiaque. Ce concept a vu le jour avec les travaux d'Otto Franck décrivant les boucles Pression/Volume ventriculaire (Fig. 1). Reprenant et poursuivant les travaux de Franck, Suga et son équipe ont défini l'élastance myocardique (Ees) et l'élastance artérielle (Ea) [3]. Ees reflète la fonction contractile du myocarde et Ea définit les propriétés viscoélastiques artérielles, en intégrant la notion de post-charge ventriculaire, composée des résistances vasculaires, de la compliance artérielle, et de l'impédance d'entrée de l'aorte. Le couplage ventriculo-aortique (Cv-a) est alors quantifié par le ratio Ea/Ees, avec un couplage adéquat défini par Ea/Ees $\cong$ 0,8, et pathologique quand Ea/Ees > 1. De plus, l'aire de la courbe Pression/Volume ventriculaire (PVA) est proportionnelle au travail ventriculaire réalisé par le coeur au cours d'un cycle cardiaque et à la consommation en oxygène du myocarde [3]. Nozawa et al. [6] ont également étudié ce sujet. Ainsi, l'analyse de la boucle Pression/Volume cardiaque permet une estimation complète des paramètres hémodynamiques. Cependant, ce concept n'a jamais été mis en place en pratique clinique du fait de la difficulté d'obtenir la courbe de pression artérielle centrale associée à la mesure simultanée des volumes ventriculaires.

Récemment, une méthode utilisant des outils déjà couramment utilisés en clinique pour suivre l'état des patients au cours des réanimations cardio-vasculaires a été décrite [4]. Cette méthode intègre les informations de pression artérielle (obtenues par un cathéter artériel radial) et de débit aortique (obtenues par Doppler trans-oesophagien), en analysant, en temps réel : (i) les boucles (*PQ loops*) représentant le débit en fonction de la pression (un cycle cardiaque correspondant à une boucle) et (ii) l'impédance vasculaire artérielle. Les auteurs de cette publication proposent une définition "fonctionnelle" de l'efficience mécanique cardiaque, correspondant au débit divisé par l'aire de la boucle. Si cette méthode est immédiatement applicable en clinique, elle reste toutefois insuffisamment informative. En effet, l'analyse de la surface totale de la boucle pression-débit ne suffit pas pour une mesurer le couplage ventriculo-aortique. En particulier, elle ne renseigne que très imparfaitement sur la rigidité artérielle et sur l'inotropisme du patient. Les auteurs de cette publication ont donc couplé l'analyse de la boucle débit/pression au calcul de l'impédance vasculaire. Toutefois, ce paramètre, décrit depuis longtemps et dont le calcul et l'analyse sont complètement indépendants de la boucle débit/pression, n'a jamais été utilisé par les praticiens du fait de la complexité de son analyse.

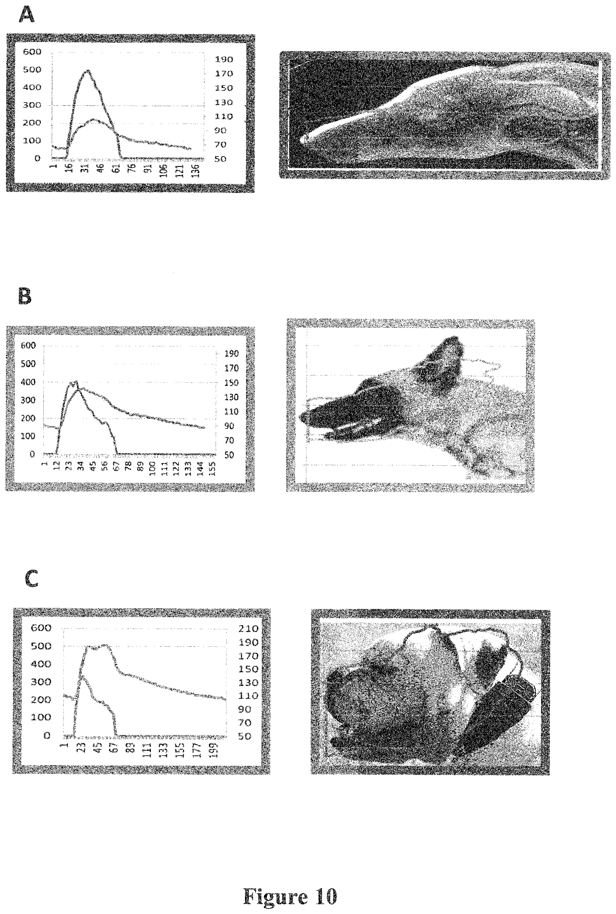

Dans une démarche initialement similaire à celle de Thiele et al., les inventeurs ont choisi d'utiliser les technologies disponibles en clinique (en particulier, la technologie du Doppler trans-oesophagien et la mesure de la pression artérielle) pour construire une boucle Pression/Débit aortique (et non Pression/Volume ventriculaire). Par une analyse de cette boucle différente de celle proposée par Thiele et al., la présente invention propose une méthode de suivi du couplage ventriculo-aortique d'un patient, qui permet d'apporter au clinicien, de façon visuelle, rapide et continue, une information précieuse sur, notamment, la rigidité artérielle du patient et sa réponse à un traitement vasoactif. La présente invention combine donc les avantages des différentes méthodes décrites précédemment, puisqu'elle allie la richesse d'information des méthodes basées sur l'analyse des courbes Pression/Volume décrites par Burkhoff et al. [3] et qu'elle peut être mise en oeuvre en utilisant les moniteurs de débit cardiaque (Doppler oesophagien ou autre) ainsi que les scopes multiparamétriques disponibles au bloc opératoire, en réanimation et dans les secteurs d'urgence. En outre, elle est utilisable en clinique de façon très visuelle et intuitive, puisque le praticien peut apprécier directement, par l'allure de la courbe, la qualité du couplage ventriculo-aortique du patient (ceci est illustré notamment à la Fig. 10).

En outre, des méthodes et des dispositifs pour mesurer des paramètres cardiaques ont été proposés dans WO 03/037181 A2 ou US 2007/0161914 A1.

Un premier aspect décrit dans le présent texte est donc une méthode pour évaluer le couplage ventriculo-aortique d'un patient en temps réel à partir de la boucle pression / flux aortique dudit patient, comprenant les étapes suivantes :

(i) déterminer, sur la boucle pression / flux, les coordonnées des points suivants :

A : point de la boucle où le flux est nul et la pression minimale (point juste avant le début de la systole : Pression diastolique),
B : point de la boucle où le flux est le plus important,
C : point de la boucle où la pression est la plus grande (pression systolique),
D : point de la boucle où le flux est nul et la pression maximale (pression dicrote, à la fermeture des valves aortiques : début de la diastole) ;

(ii) calculer le pourcentage de l'aire (Stot) de la boucle de la partie (S2) située entre les droites (AB) et (AC) et/ou l'angle $\gamma$ entre ces deux droites et/ou l'angle aigu $\delta$ entre les droites (AC) et (BD) ;
(iii) interpréter les résultats de la façon suivante :

- si S2/Stot $\leq$ R2b et/ou $\gamma \leq$ Gb et/ou $\delta \leq$ Db, le patient présente un bon couplage ventriculo-artériel ;
- si S2/Stot $\geq$ R2m et/ou $\gamma \geq$ Gm et/ou $\delta \geq$ Dm, le patient présente un mauvais couplage ventriculo-artériel ;
- dans une situation intermédiaire, la qualité du couplage ventriculo-artériel du patient est intermédiaire,

R2b, R2m, Gb, Gm, Db et Dm étant des seuils prédéterminés.
Dans ce qui précède, le "flux" désigne, de façon générale, le déplacement de la colonne de sang dans l'aorte. Il peut être mesuré, comme cela est le cas actuellement par les appareils Doppler, comme un débit (en ml/s). Toutefois, comme le débit aortique est calculé en multipliant la vélocité du sang dans l'aorte par la section de l'aorte, qui est un chiffre fixe donné selon des abaques, la méthode est également applicable en mesurant le flux comme la vitesse de la colonne de sang. Dans le présent texte, le flux désigne donc, indifféremment, un débit ou une vitesse/vélocité, et tout le raisonnement décrit dans la partie expérimentale ci-dessous peut être transposé à l'analyse de la boucle Pression/Vitesse ou Pression/Vélocité (en prenant la vitesse/vélocité comme axe des abscisses, en m/s ou cm/s). Les seuils et valeurs des paramètres angulaires seront alors différents. De même, les résultats expérimentaux décrits notamment à l'exemple 7 ci-dessous, utilisant des diagrammes de puissance avec en abscisse des vélocités et en ordonnées la pression, et figurant des courbes iso-puissance en W/cm$^2$, peuvent être transposés sur des diagrammes pression / flux, avec des courbes iso-puissance en W.
L'homme du métier pourra déterminer, en fonction des paramétrages (échelles) de la boucle obtenue avec un dispositif donné, les rapports seuils R2b et R2m et les angles seuils Gb, Gm, Db et Dm. Dans les exemples expérimentaux ci-dessous, où les boucles sont représentées en choisissant un ratio entre les échelles en ordonnée et en abscisse tel que 20mmHg et 100ml/s sont représentés par la même distance, Gb = 5° et Gm = 15°. Les valeurs seuils pour l'angle delta peuvent également être fixées, à titre d'exemple, de la façon suivante : Db = 10° et Dm = 30°. Des valeurs seuils du même ordre pourront être obtenues avec une mesure du flux en vitesse en choisissant un ratio entre les échelles en ordonnée et en abscisse tel que 1mmHg et 1 cm/s sont représentés par la même distance. Dans les exemples 1 à 4, où la pression artérielle est mesurée en périphérique et utilisée sans fonction de transfert, des valeurs seuils pour le rapport S2/Stot de l'ordre de R2b=15% et R2m= entre 40% et 50% ont été définies. L'homme du métier est parfaitement à même de redéfinir ces valeurs seuils en utilisant une fonction de transfert dont le principe est décrit à l'exemple 8. Bien évidemment, R2b < R2m, Gb < Gm et Db < Dm.
Selon une mise en oeuvre particulière de la méthode décrite dans le présent texte, la surface S1, située entre la droite (AB) et la partie inférieure de la boucle, est également calculée (par exemple, en pourcentage de la surface totale de la boucle). Cette surface donne l'information suivante (laquelle se rajoute, pour l'interprétation, à celle de S2 et/ou $\gamma$ et/ou $\delta$) :

- S1/Stot $\geq$ R1m indique que le patient a un bon couplage ventriculo-artériel ;
- S1/Stot $\leq$ R1b indique que le patient a un mauvais couplage ventriculo-artériel ;
- dans une situation intermédiaire, la qualité du couplage ventriculo-artériel du patient est intermédiaire,

R1b et R1m étant des seuils prédéterminés.
Selon une autre mise en oeuvre particulière de la méthode décrite dans le présent texte, on mesure également l'angle $\alpha$ formé par la droite (AB) et l'axe des flux et/ou l'angle $\beta$ formé par la droite (AC) et l'axe des flux. Ces angles fournissent les indications suivantes (qui s'ajoutent à celles obtenues au moins par S2 et/ou $\gamma$ et/ou $\delta$) :

- $\alpha \leq$ Ab et/ou $\beta \leq$ Bb indiquent que le patient a un bon couplage ventriculo-artériel ;
- $\alpha \geq$ Am et/ou $\beta \geq$ Bm indiquent que le patient a un mauvais couplage ventriculo-artériel ;

- dans une situation intermédiaire, la qualité du couplage ventriculo-artériel du patient est intermédiaire.

De même que pour Gb, Gm, Db et Dm ci-dessus, les valeurs seuils R1b, R1m, Ab, Am, Bb et Bm peuvent être aisément déterminées par l'homme du métier en fonction du matériel et des échelles qu'il utilise, à partir de mesures sur des patients ou sur des individus témoins dont il connaît la qualité du couplage ventriculo-artériel. La détermination des valeurs seuils est un simple travail d'"étalonnage" pour l'homme du métier. L'angle $\alpha$ fournit une information sur l'inotropisme, alors que $\beta$, qui est la somme de $\alpha$ et $\gamma$, fournit également une information sur la rigidité artérielle (comme $\gamma$).

Les méthodes décrites dans le présent texte peuvent également comprendre, à l'étape (ii), le calcul de l'aire Stot de la boucle et, à l'étape (iii) l'interprétation de Stot comme reflétant l'efficience du système cardiovasculaire (cette efficience étant d'autant plus grande que cette surface est faible). Ici encore, l'homme du métier pourra définir, avec le matériel dont il dispose et les unités qu'il utilise (notamment pour la mesure du flux, en ml/s ou en m/s), une ou plusieurs valeurs seuils pour interpréter la mesure de Stot.

Selon une mise en oeuvre simplifiée de la méthode décrite dans le présent texte, il est possible de mesurer les paramètres angulaires décrits ci-dessus à partir des seuls points A, B, C et D. Selon cette variante, seuls ces points, ainsi que les droites reliant deux de ces points, sont visualisés.

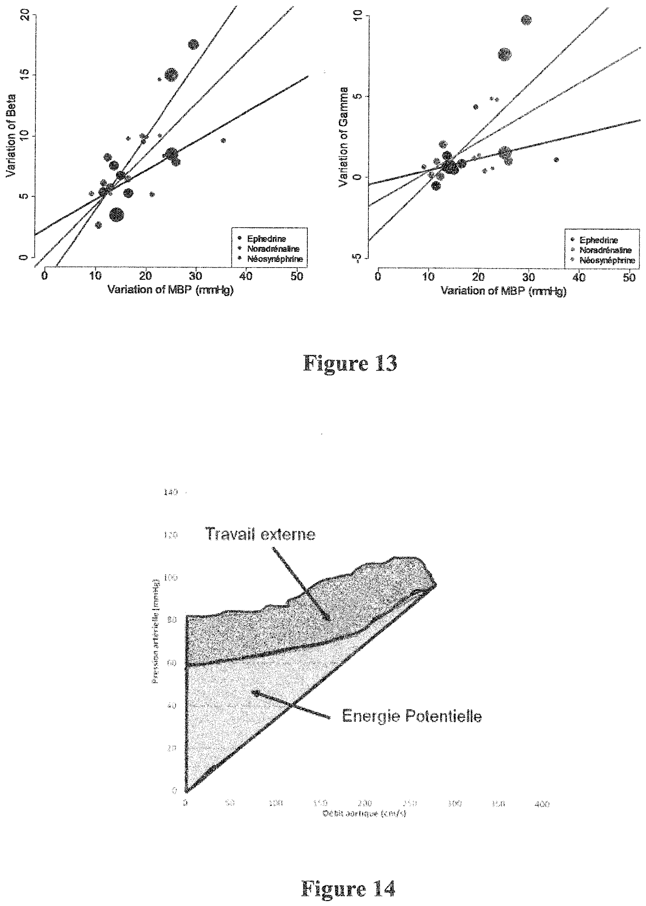

Bien évidemment, d'autres paramètres des boucles pression/flux peuvent être analysés lors de la mise en oeuvre des méthodes décrites dans le présent texte. En particulier, à partir de la première partie de la boucle (pente initiale de la boucle - proche de la pente Ees décrite par Franck et al -, aire S1 et le débit Max), on peut mesurer des paramètres reliés à l'inotropisme et la contractilité. De même, l'aire de la boucle Pression/Débit peut être utilisée pour évaluer le travail d'éjection systolique fourni par le coeur à chaque battement, à l'image des travaux de Suga et Sagawa. La surface de la courbe jusqu'à l'axe des abscisses (Fig. 14) peut également être calculée, pour évaluer l'énergie potentielle ventriculaire qui, rapportée à la surface de la boucle, permettrait une estimation de l'efficience du système cardio-vasculaire.

Il est important de noter que, contrairement à la méthode décrite par Thiele et al. (*supra*), la méthode ne nécessite pas de calculer le spectre d'amplitude de l'impédance vasculaire artérielle. En effet, les informations fournies par les différents paramètres mesurés, en particulier le pourcentage de la surface de la boucle située entre les droite (AB) et (AC) ainsi que les angles, suffisent à évaluer précisément le couplage ventriculo-artériel du patient.

Selon une mise en oeuvre particulière de la méthode décrite dans le présent texte, la puissance maximale générée par le système cardio-vasculaire du patient est calculée. La puissance est égale au produit de la pression par le débit/flux, et peut également être calculée en multipliant la pression par la vélocité (il s'agit alors d'une puissance par unité de surface). Sur le diagramme de la boucle Pression / Vélocité (Pre / Vél), on peut donc tracer des courbes Iso-Puissance liant les points où le produit Pre x Vél est identique. Selon une mise en oeuvre préférée, la boucle Pre / Vel est visualisée sur un diagramme sur lequel figurent des courbes iso-puissance (en $W/cm^2$). La puissance maximale générée par le patient peut alors être immédiatement visualisée. Un régionnement du plan du diagramme permet d'apprécier visuellement, de façon immédiate, le profil cardio-vasculaire du patient.

Ainsi, les méthodes décrites plus haut peuvent également comporter une étape d'analyse de la puissance générée par le système cardio-vasculaire du patient qui peut être effectuée de la façon suivante :

(i) à partir de la boucle pression / flux aortique du patient, déterminer la puissance maximale par $cm^2$ ($Pui_{Max}$) générée par le coeur du patient au cours d'un battement cardiaque, et les valeurs de pression (Pre) et de vélocité (Vel) au point correspondant à la valeur maximale de la puissance ;
(ii) classer le patient dans une catégorie selon les valeurs de $Pui_{Max}$, Pre et Vel déterminées à l'étape (i), en distinguant les catgories suivantes :

- 1 : $Pre > Pre_2$ et $Pre > a \times Vel$ et $Pui_{Max} \leq Pui_2$ ;
- 2 : $Pui_{Max} > Pui_2$ ;
- 3 : $Vel > Vel_1$ et $Pre \leq Pre_1$ et $Pre < b \times Vel$ et $Pui_{Max} \geq Pui_1$ ;
- 4 : $Vel > Vel_1$ et $Pre \leq Pre_1$ et $Pui_{Max} < Pui_1$ ;
- 5 : $Vel \leq Vel_1$ et $Pre \leq Pre_1$ et $Pui_{Max} < Pui_1$ ;
- 6 : $b \times Vel \leq Pre \leq a \times Vel$ et $Pui_1 \leq Pui_{Max} \leq Pui_2$ et $Pre_1 \leq Pre \leq Pre_2$ et $Vel_1 \leq Vel \leq Vel_2$ ;
- 7 : autres situations ;

les paramètres $Pre_1$, $Pre_2$, $Vel_1$, $Vel_2$, Pui1, $Pui_2$, a et b étant prédéterminés et définis de la façon suivante :

- $Pre_1$ et $Pre_2$ sont des valeurs de référence correspondant à des limites inférieure et supérieure, respectivement, d'une pression physiologique ;
- $Vel_1$ correspond à une valeur de la vélocité maximale systolique en-deçà de laquelle la vélocité maximale systolique est considérée comme inférieure à la normale ;

- Vel$_2$ correspond à une valeur de la vélocité maximale systolique au dessus de laquelle la vélocité maximale systolique est considérée comme supérieure à la normale ;
- Pui$_1$ et Pui$_2$ correspondent à des valeurs limites de la puissance maximale par cm$^2$ générée au cours d'un battement en-deçà et au-delà desquelles, respectivement, des complications peuvent survenir ;
- a et b sont des facteurs permettant d'apprécier le couplage ventriculo-artériel d'un patient, le couplage étant considéré comme satisfaisant si, au moment où la puissance générée par le coeur est maximale, b x Vel ≤ Pre ≤ a x Vel.

Les méthodes décrites ci-dessus permettent avantageusement d'identifier un patient à risque cardiovasculaire. En effet, un mauvais couplage ventricule-artériel est indicatif d'un patient à risque cardiovasculaire.

Selon un autre aspect avantageux, les méthodes décrites plus haut sont utilisées pour déterminer l'efficacité d'un traitement vasoactif pour un patient en réanimation cardiovasculaire. Pour cela, le couplage ventriculo-artériel du patient est évalué par une méthode selon l'invention, avant et après l'administration du traitement, et les résultats obtenus sont comparés. Un traitement vasoactif est considéré comme satisfaisant s'il permet la restauration d'une pression artérielle suffisante, sans toutefois entraîner une trop grande augmentation de la rigidité artérielle, reflétée par l'augmentation de S2 ,γ et δ.

Selon un autre aspect, les méthodes d'évaluation du couplage ventriculo-artériel décrites ci-dessus sont utilisées pour comparer les effets de différents traitements vasoactifs, soit pour un patient donné, pour choisir celui qui lui convient le mieux en fonction de ses caractéristiques individuelles, soit de manière plus générale, sur une ou plusieurs cohortes représentatives de patients pour qui ce type de traitement est nécessaire, en vue par exemple de fournir des recommandations. Selon cet aspect, une méthode pour comparer les effets de différents traitements vasoactifs comprend l'évaluation (par une méthode telle que décrite plus haut) du couplage ventriculo-artériel de patients recevant les traitements à comparer, avant et après chaque administration d'un des traitements. Les résultats sont ensuite analysés selon les pratiques courantes pour ce type de comparaison.

La présente invention porte sur un dispositif pour évaluer le couplage ventriculo-artériel d'un patient en temps réel, par exemple en mettant en oeuvre une méthode telle que décrite ci-dessus, caractérisé en ce qu'il comprend des moyens informatiques paramétrés pour :

(i) recevoir et traiter, en temps réel, des signaux provenant, d'une part, de moyens de mesure du flux sanguin artériel et, d'autre part, de moyens de mesure de la pression artérielle ;
(ii) afficher, en temps réel, le tracé d'au moins une boucle pression / flux artériel ;
(iii) déterminer, sur la dernière boucle pression / flux complétée, les coordonnées des points suivants :

A : point de la boucle où le flux est nul et la pression minimale,
B : point de la boucle où le flux est le plus important,
C : point de la boucle où la pression est la plus grande,
D : point de la boucle où le flux est nul et la pression maximale ; et

(iv) calculer les paramètres décrits plus haut, et en particulier le pourcentage de l'aire de la boucle (Stot) occupé par la partie S2 située entre les droites (AB) et (AC) et/ou l'angle γ entre ces deux droites et/ou l'angle aigu δ entre les droites (AC) et (BD)
(v) interpréter les résultats de la façon suivante :

si S2/Stot ≤ R2b et/ou γ ≤ Gb et/ou δ ≤ Db, le patient présente un bon couplage ventricule artériel ;
si S2/Stot ≥ R2m et/ou γ ≥ Gm et/ou δ ≥ Dm, le patient présente un mauvais couplage ventricule artériel ;
dans une situation intermédiaire, la qualité du couplage ventricule artériel du patient est intermédiaire,

R2b, R2m, Gb, Gm, Db et Dm étant des seuils prédéterminés.

Il est à noter que dans la partie expérimentale présentée ci-dessous, les inventeurs ont utilisé, pour la mesure du débit aortique, le Doppler oesophagien, mais que d'autres dispositifs de monitorage Doppler peuvent être utilisés pour cela, comme le Doppler sus-sternal ou l'échographie cardiaque, qu'elle soit trans-thoracique, trans-oesophagienne ou autre. Ces systèmes alternatifs peuvent donc, bien évidemment être utilisés avec les dispositifs selon l'invention (ou intégrés à ces dispositifs).

De même, les inventeurs ont utilisé des cathéters invasifs de mesure de la pression artérielle (en radial ou en fémoral). Ces moyens pourraient toutefois être remplacés par des moyens mesurant la pression artérielle de manière peu voire non invasive (type tonomètre, Nexfin *etc.*), qui peuvent donc également être utilisés avec les dispositifs selon l'invention (ou intégrés à ces dispositifs).

Par ailleurs, la forme de la courbe de pression étant différente en radial et en fémoral par rapport à la pression centrale aortique, l'utilisation de la courbe de pression centrale serait plus juste pour évaluer la rigidité aortique et le travail myocardique. L'obtention de cette courbe peut être réalisée aisément par l'application d'une fonction mathématique de transfert à partir de la courbe de pression radiale ou fémorale. Selon une réalisation particulière du dispositif selon l'invention, les moyens informatiques sont également paramétrés pour calculer la pression centrale aortique, à partir de la pression artérielle mesurée.

Selon une autre mise en oeuvre du dispositif de l'invention, les moyens informatiques sont également paramétrés pour afficher l'évolution de S2 et/ou de δ en fonction du temps. Ceci peut être utile pour assurer un meilleur suivi du patient sur la durée et pour conserver un historique de son couplage ventriculo-aortique et de ses réactions aux différents traitements vasoactifs qu'il aura reçus.

Selon une autre mise en oeuvre du dispositif de l'invention, les moyens informatiques sont également paramétrés pour afficher des courbes iso-puissance.

Le dispositif de l'invention peut également comprendre des moyens informatiques sont paramétrés pour afficher le tracé d'une boucle pression / flux artériel en fonction du temps, le temps étant représenté par un troisième axe.

Le présent texte décrit en outre une méthode pour obtenir une information sur l'état cardio-vasculaire d'un patient, comportant les étapes suivantes :

(i) à partir de la boucle pression / flux aortique du patient, déterminer la puissance maximale par $cm^2$ ($Pui_{Max}$) générée par le système cardivasculaire du patient au cours d'un battement cardiaque, et les valeurs de pression (Pre) et de vélocité (Vel) au point correspondant à la valeur maximale de la puissance ;

(ii) classer le patient dans une catégorie selon les valeurs de $Pui_{Max}$, Pre et Vel déterminées à l'étape (i), en distinguant les catgories suivantes :

- 1 : $Pre > Pre_2$ et $Pre > a \times Vel$ et $Pui_{Max} \leq Pui_2$ ;
- 2 : $Pui_{Max} > Pui_2$ ;
- 3 : $Vel > Vel_1$ et $Pre \leq Pre_1$ et $Pre < b \times Vel$ et $Pui_{Max} \geq Pui_1$ ;
- 4 : $Vel > Vel_1$ et $Pre \leq Pre_1$ et $Pui_{Max} < Pui_1$ ;
- 5 : $Vel \leq Vel_1$ et $Pre \leq Pre_1$ et $Pui_{Max} < Pui_1$ ;
- 6 : $b \times Vel \leq Pre \leq a \times Vel$ et $Pui_1 \leq Pui_{Max} \leq Pui_2$ et $Pre_1 \leq Pre \leq Pre_2$ et $Vel_1 \leq Vel \leq Vel_2$ ;
- 7 : autres situations ;

les paramètres $Pre_1$, $Pre_2$, $Vel_1$, $Vel_2$, Pui1, $Pui_2$, a et b étant prédéterminés et définis de la façon suivante :

- $Pre_1$ et $Pre_2$ sont des valeurs de référence correspondant à des limites inférieure et supérieure, respectivement, d'une pression physiologique ;
- $Vel_1$ correspond à une valeur de la vélocité maximale systolique en-deçà de laquelle la vélocité maximale systolique est considérée comme inférieure à la normale ;
- $Vel_2$ correspond à une valeur de la vélocité maximale systolique au dessus de laquelle la vélocité maximale systolique est considérée comme supérieure à la normale ;
- $Pui_1$ et $Pui_2$ correspondent à des valeurs limites de la puissance maximale par $cm^2$ générée au cours d'un battement en-deçà et au-delà desquelles, respectivement, des complications peuvent survenir ;
- a et b sont des facteurs permettant d'apprécier le couplage ventriculo-artériel d'un patient, le couplage étant considéré comme satisfaisant si, au moment où la puissance générée par le coeur est maximale, $b \times Vel \leq Pre \leq a \times Vel$ ;

(iii) déduire l'information suivante sur l'état cardio-vasculaire du patient :

- si le patient a été classé dans la catérogie 1, il présente un risque d'hypertension artérielle gênant l'éjection cardiaque ;
- si le patient a été classé dans la catégorie 2, il se trouve dans un état supra-physiologique induisant un risque de rupture capillaire ;
- si le patient a été classé dans la catégorie 3, il présente un risque d'hypoperfusion ;
- si le patient a été classé dans la catégorie 4, il présente un risque d'insuffisance circulatoire ;
- si le patient a été classé dans la catégorie 5, il présente une insuffisance circulatoire sévère ;
- si le patient a été classé dans la catégorie 6, il se trouve dans un état physiologique avec une puissance et un couplage ventriculo-artériel satisfaisants ;
- les autres situations correspondant à des états intermédiaires entre ceux des catégories 1 à 6.

A titre d'exemple non limitatif, les paramètres $Pre_1$, $Pre_2$, $Vel_1$, $Vel_2$, Pui1, $Pui_2$, a et b ont été définis, dans l'exemple 7 ci-dessous, de façon ce que $Pre_1 \times Vel_1 = Pui_1$ et $Pre_2 \times Vel_2 = Pui_2$. Dans cet exemple, les valeurs nunmériques de ces paramètres sont :

$Pre_1$ = 60 mmHg = $0,798 \times 10^4$ Pa ;
$Pre_2$ = 160 mmHg = $2,128 \times 10^4$ Pa ;
$Vel_1$ = 60 cm/s ;
$Vel_2$ = 160 cm/s ;
a=2 ;
b=0 ,5 ;
$Pui_1$=0,48 $W/cm^2$ ; et
$Pui_2$=3,4 $W/cm^2$.

Le présent texte décrit en outre une méthode théranostique pour aider à la décision thérapeutique concernant un patient à risque cardio-vasculaire, comportant les étapes suivantes :

(i) obtenir une information sur l'état cardio-vasculaire d'un patient par la méthode ci-dessus ; et
(ii) déduire le type de traitement préconisé pour le patient selon la catégorie dans laquelle il a été classé.

Dans une mise en oeuvre préférée de cette méthode, le traitement préconisé, à l'étape (ii) est préférentiellement choisi parmi :

- pour un patient classé dans la catégorie 1 : les antihypertenseurs vasodilatateurs, les diurétiques, les bêta-bloquants et leurs combinaisons ;
- pour un patient classé dans la catégorie 2 : les hypnotiques, les morphinomimétiques et les antihypertenseurs ;
- pour un patient classé dans la catégorie 3 : les vasoconstricteurs ;
- pour un patient classé dans la catégorie 4 : les inotropes positifs, l'expansion volémique et leur combinaison ;
- pour un patient classé dans la catégorie 5 : l'expansion volémique couplée à un vasoconstricteur et les inotropes positifs ;

-- pour un patient classé dans la catégorie 7 : les solutés de remplissage, les inotropes et leur combinaison en cas de vélocité basse et les bêtabloquants en cas de vélocité élevée.

Le présent texte décrit en outre une méthode de traitement d'un patient à risque cardio-vasculaire, comportant les étapes suivantes :

(i) déterminer le type de médicament préconisé pour le patient par une méthode théranostique telle que décrite ci-dessus ; et
(ii) administrer un traitement au patient, ledit traitement étant choisi parmi les traitements préconisés.

Dans une mise en oeuvre préférée des méthodes ci-dessus impliquant des diagrammes de puissance, la boucle pression / vélocité est visualisée sur un digramme de puissance sur lequel figurent également au moins les courbes iso-puissance $Pui_1$ et $Pui_2$ et les droites de découplage Pre = a Vel et Pre = b Vel.

Les exemples et figures suivants illustrent l'invention sans toutefois limiter son étendue.

### Légende des figures :

Figure 1 : Figure 1 : Schéma d'une boucle pression-volume au niveau du ventricule gauche (VG : ventricule gauche. PTS : pression télé-systolique. PTD : pression télé-diastolique. VTS : volume télé-systolique. VTD : volume télé-diastolique, Es : élastance ventriculaire, Ea : élastance artérielle). Point A : ouverture de la valve mitrale, point B : Fermeture de la valve mitrale, point C : ouverture de la valve Aortique, point D : fermeture de la valve aortique.
Figure 2 : Courbes de pression et de débit, en fonction du temps.
Figure 3 : Boucle Pression/Débit - définition des points remarquables.
Figure 4 : Correspondance des points de la boucle P/D dans le domaine temporel.
Figure 5 : Les surfaces de la boucle Pression/Débit.
Figure 6 : Les angles de la boucle Pression/Débit.

Figure 7 : exemple de courbe pression-débit pour un patient présentant un bon couplage.

Figure 8 : exemple de courbe pression-débit pour un patient présentant un mauvais couplage.

Figure 9 : exemples de de courbes pression-débit pour des chiens de 3 races très différentes.

Figure 10 : A : Bon couplage ventriculo-aortique: Profil de lévrier ; B : Couplage ventriculo-aortique intermédiare: Profil de berger allemand ; C : Mauvais couplage ventriculo-aortique: Profil de bulldog.

Figure 11 : Evolution des ondes de pression périphérique (en haut) et centrale (en bas), chez le sujet sain (à gauche) et le sujet souffrant d'artériosclérose (à droite). Chez le sujet sain, la résultante des pressions incidente et réfléchie n'augmente pas la pression systolique : ici l'Aix mesuré est de 91%. Chez le patient à risque à droite, l'augmentation de rigidité artérielle est responsable de la précocité des ondes de réflexions qui amplifie la pression systolique centrale, l'Aix résultante est de 126%.

Figure 12 : Corrélation entre Central Aix et les paramètres de la boucle.

Figure 13 : Comparaison de l'évolution de la boucle P/D en fonction du vasoconstricteur utilisé.

Figure 14 : Signification de l'aire de la boucle (Stot) et visualisation de l'énergie potentielle permettant de calculer l'efficience du système.

Figure 15 : Effet visuel du changement d'unité en vélocité sur la forme et les paramètres issus de la boucle. A : Boucle Pre/Vel chez un sujet jeune. B : Boucle P/D chez un sujet jeune. C : Boucle Pre/Vel chez un sujet âgé. D : Boucle P/D chez un sujet âgé

Figure 16 : Exemple de boucle Pre/Vel obtenu avec un autre dispositif de mesure de vélocité aortique. A.B. Mesures obtenues avec un doppler sus-sternal. C.D. Mesures obtenues avec un echographe trans-thoracique disposant d'un doppler pulsé sur lequel le tir doppler est positionné sur la chambre de chasse aortique.

Figure 17 : Exemple de boucle Pre/Vel obtenue de façon entièrement non-invasive, la pression étant recueillie au moyen d'un capteur de pression digitale Clearsight, la vélocité étant recueillie au moyen d'un échographie trans-thoracique.

Figure 18 : Représentation de la boucle Pre/Vel en quadrilataire.

Figure 19 : Représentation de la boucle Pre/Vel en trois dimension, en prenant en compte le temps sur un troisième axe.

Figure 20 : Diagramme de puissance et description des isopuissances.

Figure 21 : Diagramme de puissance et description des axes de couplage et de découplage.

Figure 22 : Interprétation physiologique de la boucle Pre/Vel en relation avec le diagramme de puissance.

Figure 23 : Exemple d'utilisation de la boucle Pre/Vel et du diagramme de puissance en condition de vasodilatation excessive.

Figure 24 : Exemple d'utilisation de la boucle Pre/Vel et du diagramme de puissance en condition d'hypovolémie.

Figure 25 : Exemple d'utilisation de la boucle Pre/Vel et du diagramme de puissance en condition d'insuffisance cardiaque.

Figure 26 : Autre exemple d'utilisation de la boucle Pre/Vel et du diagramme de puissance en condition d'insuffisance cardiaque.

Figure 27 : Exemple d'utilisation de la boucle Pre/Vel et du diagramme de puissance en condition d'hypertension excessive.

Figure 28 : Illustration des différences entre pression centrale et pression périphérique, et effets sur la forme de la boucle vel/pre.

Figure 29 : Exemple du résultat de l'application d'une fonction de transfert à un signal de pression périphérique et comparaison avec la pression centrale mesurée.

## EXEMPLES

## Exemple 1 : Construction de la boucle Pression/Débit aortique

### 1.1 - Paramètres mesurés

- Le signal de Pression est collecté en mm Hg (Fig. 2), par un cathéter artériel radial ou fémoral.
- Le signal Doppler oesophagien donnant le débit aortique en ml/s (Fig. 2) est collecté via le moniteur combi Q Deltex®.
- La boucle Pression/Débit est construite battement par battement cardiaque avec les valeurs simultanées de débit aortique en abscisse et de pression en ordonnée.

***1.2 - Description des différents paramètres issus de la boucle Pression/Débit aortique :***

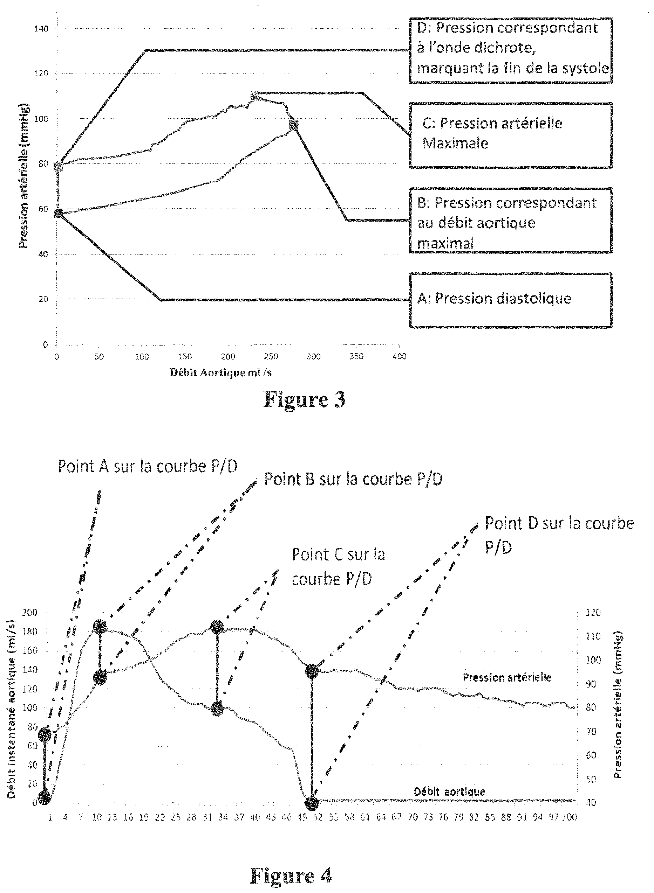

- Les différents points spécifiques de la boucle (Fig. 3-4) :

  - **A :** Pression diastolique : le débit est nul avant le début de la systole.
  - **B :** Débit maximum.
  - **C :** Pression maximun (Pression systolique).
  - **D :** Pression Dicrotique : pression à la fermeture des valves aortique : débit à 0, début de la diastole.

- Les différentes droites spécifiques de la boucle :

  - Droite passant par les points A et B : droite entre le début du cycle et le point où le débit est maximal.
  - Droite passant par les points A et C : droite entre le début du cycle et le point où la pression est maximale.

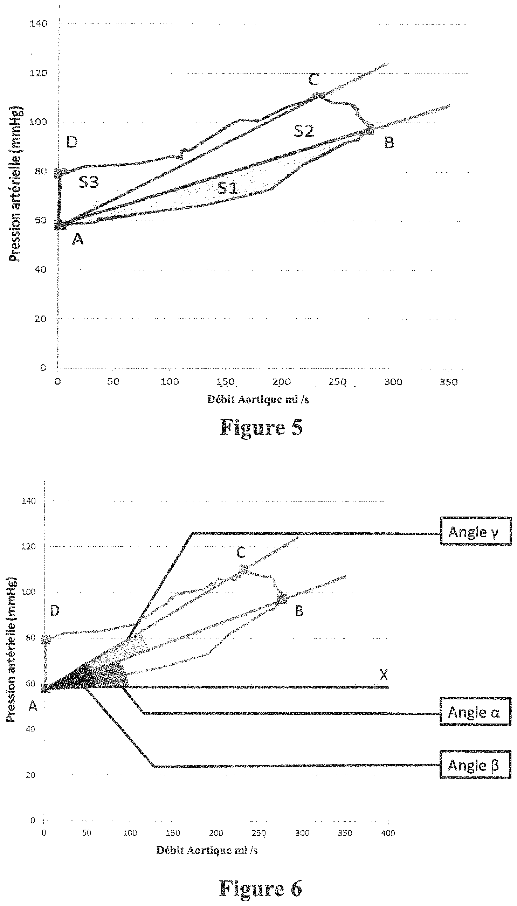

- Les différentes surfaces spécifiques de la boucle (Fig. 5) :

  - **Stot :** surface totale de la boucle Pression/débit
  - **S1** : surface entre la partie inférieure de la courbe et la droite (AB)
  - **S2 :** surface de la boucle comprise entre les deux droites (AB) et (AC)
  - **S3 :** surface de la boucle comprise entre la droite (AC) et le bord supérieur de la boucle.

- Les différents angles de la boucle (Fig. 6) :

  - **Alpha :** angle entre l'horizontale passant par le point A et la droite (AB)
  - **Beta :** angle entre l'horizontale passant par le point A et la droite (AC).
  - **Gamma :** angle entre les deux droites (AB) et (AC).

## Exemple 2 : Principe physiologique de l'analyse des différents paramètres issus de la boucle Pression/Débit aortique

Un bon couplage ventriculo-artériel entraine une augmentation de la pression artérielle lors de l'éjection ventriculaire, avec une diminution de la pression lorsque le débit d'éjection diminue : le débit et la pression vont grossièrement dans le même sens au cours de la systole. Un mauvais couplage ventriculo-artériel apparait lorsque durant la systole, la pression artérielle augmente alors que le débit d'éjection diminue. Cela arrive avec l'apparition d'ondes de réflexion précoce, favorisées par une augmentation de la rigidité aortique. Il en résulte une augmentation de la pression systolique en télé-systole avec une baisse concomitante du débit d'éjection.

La rigidité aortique augmente avec l'âge et les facteurs de risques cardiovasculaires, ainsi que par une vasoconstriction médicamenteuse excessive.

Sur la boucle Pression/Débit, ce phénomène sera représenté par une grande surface **S2** de la boucle P/D et du fait de la montée et précoce de la pression, des angles **beta** et **gamma** importants.

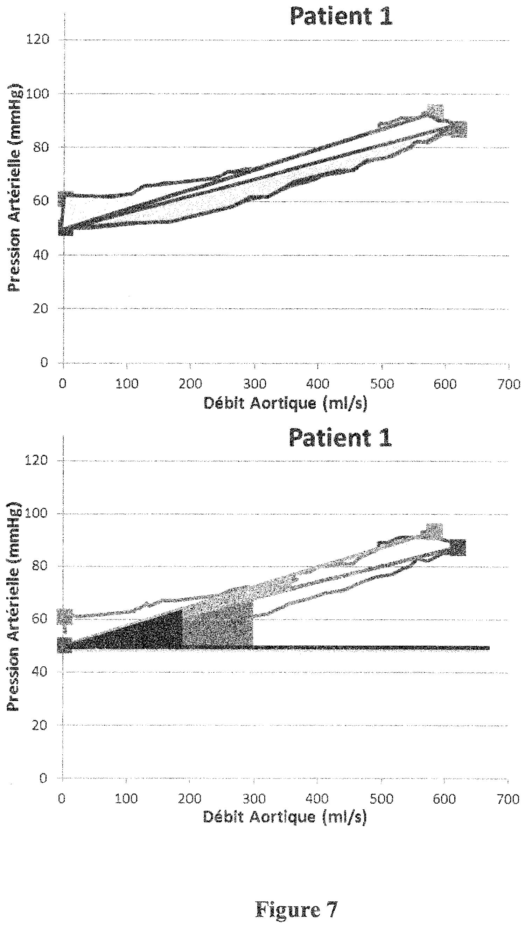

Pour un patient présentant un bon couplage ventriculo-artériel (Patient 1, Fig. 7, Tableau 1), on observe donc :

- une forme allongée de la boucle P/D ;
- une surface S1 prédominante par rapport à S2 et S3, et une surface S2 représentant un faible pourcentage de la surface totale; et
- un angle gamma presque nul.

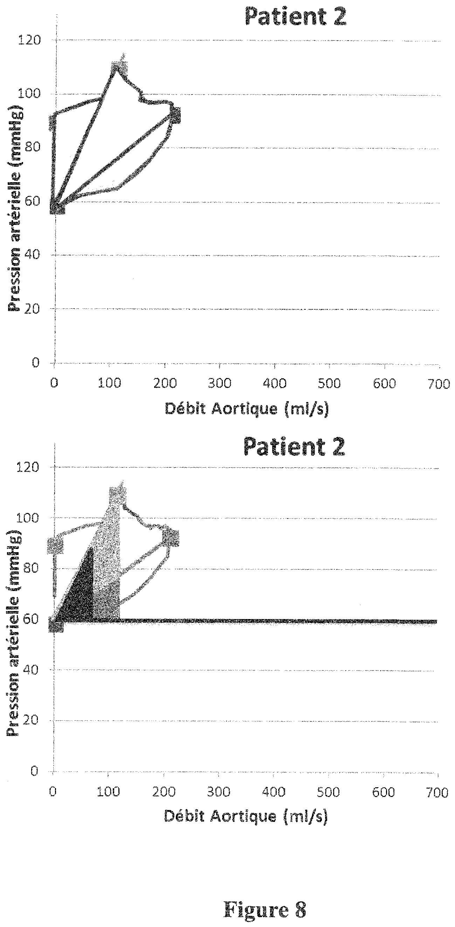

Pour un patient présentant un mauvais couplage ventriculo-artériel (Patient 2, Fig. 8, Tableau 1), on observe en revanche :

- Une forme plus tassée et carrée de la courbe P/D ;
- Une surface totale importante avec une surface S2 représentant au moins la moitié de la surface totale et une surface S3 représentant un pourcentage important de la surface totale ;
- Un angle gamma (donc également bêta) important.

**Tableau 1** : valeur des paramètres de la boucle pression/débit pour des patients représentatifs d'un bon (patient 1) ou d'un mauvais (patient 2) couplage ventriculo-artériel

| Indices | Patient 1 | Patient 2 |
|---|---|---|
| Surface totale | 5344 ml/s * mmHg | 6495 ml/s * mmHg |
| S1 | 3710 ml/s * mmHg - 69% | 1444 ml/s * mmHg - 22% |
| S2 | 484 ml/s * mmHg - 9% | 3324 ml/s * mmHg - 51% |
| S3 | 1150 ml/s * mmHg - 22% | 1727 ml/s * mmHg - 27 % |
| Alpha | 5,2° | 9,2° |
| Beta | 5,4° | 24,5° |
| Gamma | 0,2° | 15 ,2° |

Cette méthode a été mise en oeuvre sur des chiens de trois races différentes :

- le lévrier : chien longiligne, aux longues pattes fines, au corps souple et léger, très musclé, tendineux, bâti pour la course ;
- le bulldog anglais : exemple type du molosse, massif et très peu endurant à l'effort ;
- le berger allemand : chien sportif et polyvalent, moins rapide toutefois que le lévrier, intermédiaire entre les deux précédents.

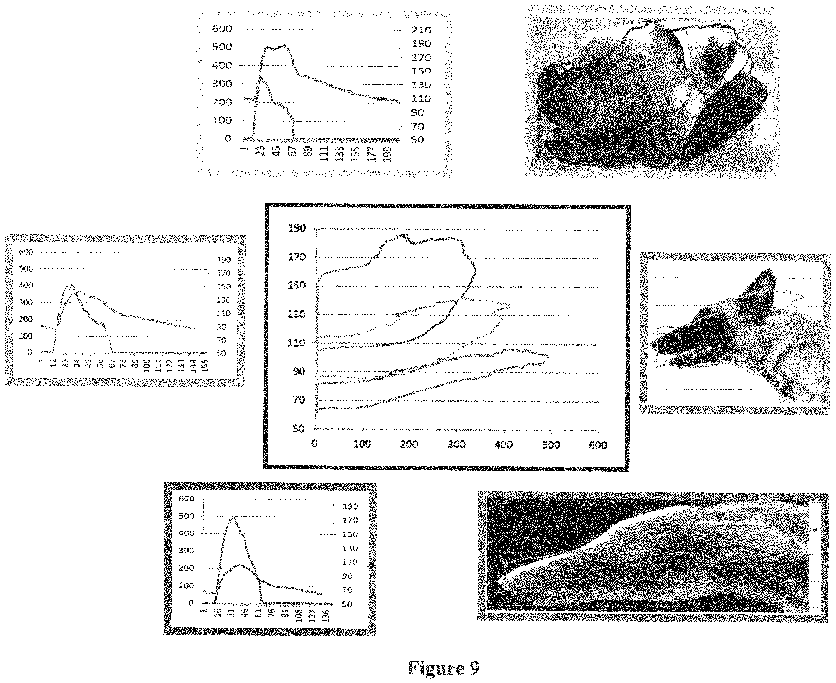

Les résultats obtenus (Fig. 9 et tableau 2) montrent 3 profils différents : le lévrier a un profil typique d'un excellent couplage ventriculo-aortique, à l'opposé de celui du bulldog, tandis que le berger allemand présente un profil intermédiaire.

**Tableau 2** : paramètres mesurés pour 3 chiens de races différentes

|  | Lévrier | Berger Allemand | Bulldog |
|---|---|---|---|
| Stot | 5200 | 6900 | 6500 |
| S1 | 40% | 35% | 22% |
| S2 | 25% | 32% | 51% |
| S3 | 35% | 33% | 27% |
| Alpha | 2.1 | 3.2 | 7.1 |
| Beta | 3.3 | 7.1 | 21 |
| Gamma | 1.2 | 3.9 | 12.9 |

Ces résultats ont conduit les inventeurs à proposer un moyen particulièrement intuitif pour reconnaître au premier coup d'oeil la qualité du couplage ventriculo-aortique d'un patient (Fig. 10) : un patient ayant un très bon couplage ventriculo-aortique aura une boucle de la forme du profil de la tête du lévrier, une boucle ressemblant davantage au profil d'une tête de berger allemand correspondra plutôt à un couplage ventriculo-aortique moyen, tandis qu'un patient ayant un mauvais couplage ventriculo-aortique sera immédiatement identifié par une boucle pression/flux ayant une forme ressemblant au profil de la tête du bulldog, massive (Fig. 10).

La représentation visuelle de la boucle Pression/Débit avec l'analyse couplée des différentes surfaces et des différents angles permettent facilement d'évaluer le couplage ventriculo-artériel des patients :

- à l'état de base, en fonction de leurs facteurs cardio-vasculaires ; et
- en suivant l'évolution en fonction de la réponse à une thérapeutique vasoactive.

**Exemple 3 : Suivi de patients et de leur réponse à une thérapeutique vasoactive, comparaison de différents médicaments vasoactifs**

*3.1 - Méthodes*

*Patients*

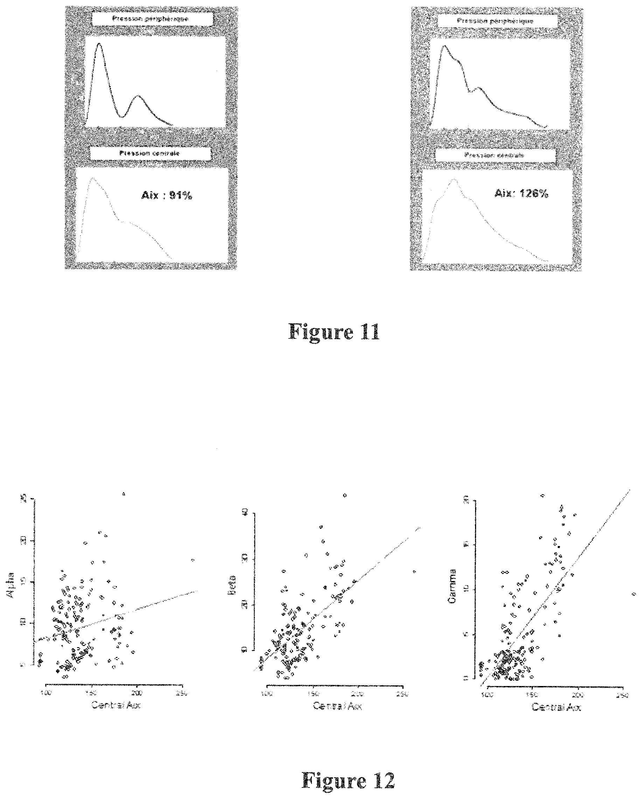

L'étude a été menée au bloc opératoire sur des patients majeurs de neurochirurgie, après accord du comité d'éthique (CE SRLF 11-356) et recueil du consentement éclairé. En plus du monitorage standard, un dispositif de mesure simultanée de la pression artérielle invasive et du débit cardiaque par Doppler trans-oesophagien (CombiQ®, Deltex Medical) était mis en place après induction de l'anesthésie générale. Par ailleurs, pour avoir un reflet de l'élastance artérielle et de la rigidité artérielle, un tonomètre artériel (SphygmoCor®, AtCor Medical) a été utilisé pour obtenir la courbe de pression artérielle centrale et les indices de rigidité artérielle. Cet appareil est actuellement la référence pour quantifier la rigidité artérielle (représentée par l'augmentation index : Aix, Fig. 11) et suivre son évolution en fonction des thérapeutiques vasoactives.

Les patients ayant des épisodes de baisse de pression artérielle (baisse de 20% par rapport à la pression cible), et nécessitant l'administration de vasoconstricteur ont été inclus. Avant chaque injection de vasopresseur (T0) puis au moment du pic d'effet du vasopresseur (T1), les boucles Pression/Débit ont été tracées et les paramètres dérivés de la boucle ont été calculés. Au même moment, à l'aide du SphygmoCor®, l'augmentation index (Aix) et l'élastance artérielle (Ea) ont été recueillis.

Les patients ont reçu, selon le choix de l'anesthésiste, soit 9mg d'Ephedrine, soit 50 $\mu$g de Néophényléphrine, soit 5 $\mu$g Noradrénaline administrée sur une voie veineuse périphérique.

*Analyse statistique:*

- Recherche de la corrélation entre l'Aix mesuré par le SphygmoCor®, avec les paramètres de la boucle P/D à T0 et à T1.
- Comparaison de l'évolution des paramètres de la boucle P/D en fonction de l'utilisation d'Ephedrine, de Néosynephrine ou de Noradrénaline.
- Comparaison des paramètres de la boucle P/D à T0 entre les patients porteurs de facteurs de risques cardiovasculaires et les patients non porteurs de facteurs de risques.
- Etude des variations des paramètres de la boucle P/D avant et après vasoconstriction en fonction du terrain du patient et du type de vasoconstricteur utilisés.

**3.2 - Corrélation des paramètres de la courbe P/D avec la rigidité artérielle, mesurée par un tonomètre artériel**

Cent quatre bolus de vasoconstricteurs (25 d'Ephedrine, 40 de Néophényléphrine et 39 de Noradrénaline), administrés à 15 patients (47 ans d'âge médian, 67 hommes/ 8 femmes) ont été étudiés. Sept patients n'avaient aucun facteur de risques cardiovasculaires (*Healthy*), et 8 patients avaient au moins un facteur de risques cardiovasculaire (*Comorbid*).

Les résultats montrent que l'Aix est très fortement corrélé à beta, gamma et S2, moyennement corrélé à Stot et S3 et très peu à alpha et S1 (Fig. 12 et tableau 3).

**Tableau 3** : Coefficient de corrélation de Pearson (IC à 95%) avec Aix

|  | Coefficient de corrélation de Pearson (IC à 95%) avec Aix |
|---|---|
| **Stot** | 0.37 [0.23 - 0.49] |
| **S1** | -0.12 [-0.27 - 0.04] |
| **S2** | 0.47 [0.34 - 0.58] |
| **S3** | 0.27 [0.12 - 0.41] |
| **Alpha** | 0.23 [0.08 - 0.37] |
| **Beta** | 0.63 [0.53 - 0.71] |
| **Gamma** | 0.67 [0.57 - 0.74] |

### 3.3 - Comparaison des effets induits par différents médicaments vasoactifs

L'Ephedrine, la Noradrénaline et la Néosynéphrine induisent majoritairement une augmentation significative des différentes surfaces (Stot, S1, S2, S3) et angles (alpha, beta et gamma) avant et après vasonconstriction (Tableau 4). Cependant, la Neosynephrine induit une augmentation plus importante que les deux autres vasoconstricteurs pour Stot, beta, gamma et S2 (en valeur absolue et en pourcentage par rapport à Stot (S2/Stot)) (Tableau 5).

**Tableau 4 :** Variation des paramètres de la boucle P/D suite à un traitement vasoactif

| EPHEDRINE | | | |
|---|---|---|---|
| | Base | Pic | p value |
| S tot | 4889 (4004 à 5807) | 6974 (5862 à 8831) | 0.0001 |
| S1 | 1997 (1644.7 à 2310.4) | 2722 (1999.3 à 3762.6) | 0.0018 |
| S2 | 1709 (833.3 à 2331.8) | 2694.4 (762.9 à 3299) | 0.0302 |
| S3 | 1658.8 (1081 à 1875.7) | 2242.5 (1590.9 à 2557.6) | 0.0014 |
| S2/Stot | 34.2 (17.7 à 40.6) | 32.6 (12.1 à 40.7) | 0.5554 |
| Alpha | 7.4 (5.1 à 9.2) | 9.5 (6.3 à 11.3) | 0.0012 |
| Beta | 10.9 (6.8 à 12.7) | 13.3 (8.4 à 19.3) | 0.0002 |
| Gamma | 1.8 (0.9 à 3.8) | 2.4 (0.9 à 5.6) | 0.055 |
| NORADRENALINE | | | |
| | Base | Pic | p value |
| S tot | 5291 (4094.5 à 6151) | 6582 (5170.5 à 7445) | 0.0037 |
| S1 | 1775 (1480.5 à 2208.4) | 1963.2 (1628.5 à 2776.1) | 0.0787 |
| S2 | 1215.9 (750 à 2504.4) | 1957.4 (1139.8 à 2614.2) | 0.0979 |
| S3 | 1860.8 (1350.8 à 2290.6) | 2311.5 (1561.8 à 2800.3) | 0.0146 |
| S2/Stot | 26.4 (17 to 39.7) | 27.9 (19.9 to 37.3) | 0.7722 |
| Alpha | 8.5 (5.2 à 11.2) | 10.6 (7.2 à 13.8) | <0.0001 |
| Beta | 11.5 (7.2 à 15.8) | 14.2 (9.8 à 22.9) | <0.0001 |
| Gamma | 2.2 (1.2 à 4) | 3.2 (1.7 à 9.3) | 0.0011 |
| NEOSYNEPHRINE | | | |
| | Base | Pic | p value |
| S tot | 5344 (4029.5 à 7087.5) | 7412 (6247.5 à 9019) | <0.0001 |
| S1 | 2028 (1437.8 à 2478.4) | 2516 (1574.5 à 3184.5) | 0.0725 |
| S2 | 1255.2 (286.4 à 2980.9) | 2624.7 (1502.6 à 3318.1) | 0.0043 |
| S3 | 1851.1 (1306.9 à 2381.5) | 2452.2 (2008.1 à 3267.5) | 0.0033 |
| S2/Stot | 26.9 (7.9 to 38.4) | 31.2 (23.5 to 41.7) | 0.165 |
| Alpha | 8.5 (5.1 à 10.4) | 10.6 (7.3 à 13.6) | <0.0001 |
| Beta | 11.2 (7.3 à 15.2) | 16.2 (11.1 à 23.6) | <0.0001 |
| Gamma | 2.1 (0.9 à 3.6) | 5.4 (2.3 à 11.5) | 0.0011 |

**Tableau 5 :** comparaison des effets de la Neosynephrine et de la Noradrénaline

| NEOSYNEPHRINE *vs* NORADRENALINE | | |
|---|---|---|
| | Variations (Diff [95% CI]) | p value |
| S tot | 1072.27 [414.96 à 1729.59] | 0.002 |
| S1 | 266.01 [-139.2 à 671.22] | 0.2022 |
| S2 | 615.97 [296.49 à 935.46] | 0.0003 |
| S3 | 221.27 [-146.76 à 589.31] | 0.2424 |
| S 1/Stot % | -4.61 [-9.76 à 0.55] | 0.084 |
| S2/Stot % | 6.6 [1.31 à 11.9] | 0.0169 |
| S3/Stot % | -2.13 [-6.37 à 2.12] | 0.3294 |
| Alpha | -0.02 [-0.72 à 0.69] | 0.9619 |
| Beta | 2.55 [1.27 à 3.82] | 0.0002 |
| Gamma | 2.59 [1.5 à 3.69] | <0.0001 |

Sur la figure 13, on remarque que la Neosynephrine augmente les angles Beta et Gamma significativement plus que la Noradrénaline et que l'Ephedrine, pour des augmentations similaires de pression induites par les vasoconstricteurs.

### 3.4 - Comparaison des paramètres de la boucle P/D entre patients porteurs ou non de facteurs de risques ainsi que de l'évolution de la boucle après vasoconstricteurs

A l'état de base, les patients présentant des facteurs de risque (*Comorbid*) ont des angles alpha, beta et gamma plus grands que les patients n'en présentant pas (*Healthy*)(Tableau 6).

**Tableau 6** : Paramètres en fonction de la présence ou non de facteurs de risques

| | Healthy | Comorbid | p value |
|---|---|---|---|
| S tot | 5374 (4360.2 à 6704) | 5047 (4006 à 6424.8) | 0.6484 |
| S1 | 2101.5 (1223.7 à 2750.2) | 1737 (1494 à 2217.8) | 0.9137 |
| S2 | 1322.1 (831.6 à 2424.3) | 1110.2 (407.7 à 2871.4) | 0.5147 |
| S3 | 1928.1 (1208.1 à 2417.8) | 1802 (1418.5 à 2271.9) | 0.8851 |
| Alpha | 5.2 (4.8 à 8.3) | 10.4 (8.6 à 12.3) | 0.0276 |
| Beta | 7.1 (6.3 à 10) | 15.7 (11.8 à 18.4) | 0.0007 |
| Gamma | 1.5 (0.9 à 2.1) | 3.7 (1.5 à 9.1) | 0.0304 |

**Conclusion:**

Cette étude montre que la représentation visuelle de la boucle Pression/Débit avec l'analyse couplée des différentes surfaces et des différents angles permet facilement d'évaluer le couplage ventriculo-artériel des patients de la manière suivante :

- La mesure de l'angle beta et/ou gamma renseigne en continu sur la rigidité aortique des patients.
- Pour la même augmentation de pression artérielle, l'évolution de la forme de la boucle P/D est différente en fonction du type de vasoconstricteurs utilisés. Bien que les angles et Stot augmentent avec tous les vasonstricteurs, l'effet de la Neosynephrine implique une augmentation de S2 et des angles beta et gamma plus importante que les autres vasoconstricteurs. Ceci reflète un effet délétère de ce médicament sur le couplage ventriculo-aortique. La Neosynephrine étant un puissant vasoconstricteur artériel, cet effet sur la post-charge ventriculaire est attendu, mais la représentation de la boucle P/D permet de le visualiser et de le quantifier directement. L'évolution de la forme de la boucle P/D permet donc de suivre et de comparer les effets des médicaments vasoactifs.

- Les angles Beta et Gamma de la boucle P/D permettent de différencier les patients porteurs ou non de facteurs de risque cardiovasculaires. La forme de la boucle permet donc d'identifier immédiatement les patients à risque cardiovasculaire.
- L'analyse seule de la surface totale Stot comme paramètre reflétant le couplage ventriculo-aortique ne suffit pas pour une analyse complète. En effet, aucune différence de Stot n'apparaît entre les patients avec ou sans facteurs de risque, et Stot augmente quel que soit le vasoconstricteur utilisé.

**Example 4 : variantes de mesure des paramètres utilisés pour construire la boucles**

*4.1 - Construction de la boucle Pression / Vélocités au lieu de la boucle Pression / Débit*

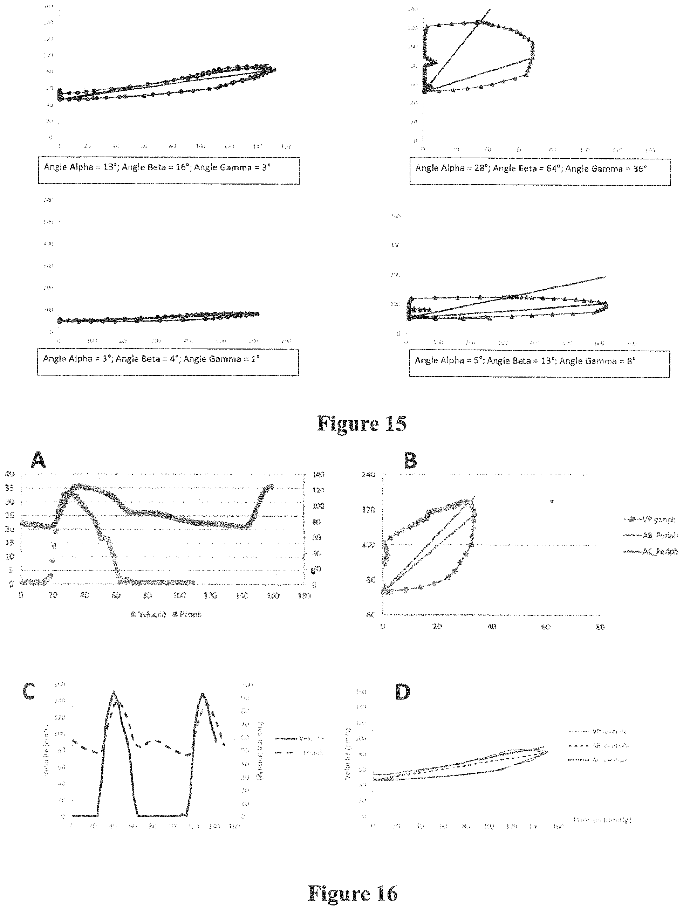

Les vélocités du sang dans l'aorte thoracique sont de l'ordre de 50 à 200 cm/s. Cette échelle de valeur numérique est dans le même ordre de grandeur que la pression exprimé en mm Hg. Cela n'est pas vrai avec le Flux (Flo), qui est représenté par la Vélocité (Vel) multiplié par la section de l'Aorte (Flo = Vel x SAo) et qui a donc une échelle de 100 à 600 ml/s. Cette particularité fait que les angles calculés avec la boucle Pression / Vélocité (ci-après désignée "boucle Pre / Vél") sont d'un ordre de grandeur entre 30 et 60° en utilisant des échelles en abscisse et ordonnée telles que 1 cm/s et 1 mm Hg sont représentés par la même distance.
La construction de la boucle Pre / Vél est donc plus visuellement accessible, compréhensible et reproductible avec les mêmes résultats statistiques du fait du facteur constant de la section de l'aorte pour le calcul du débit (Figure 15).

*4.2 - Construction de la boucle avec un Doppler Sus-sternal ou une échographie (transthoracique ou oesophagienne)*

Les boucles décrites ci-dessus ont été obtenues avec un Doppler oesophagien mesurant le débit dans l'aorte thoracique descendante. Cela peut être également fait avec d'autres appareils Doppler mesurant la vélocité du sang dans l'aorte, comme le Doppler sus-sternal et l'échographie trans-thoracique.
La figure 16 présente deux exemples réalisés avec un appareil Doppler sus-sternal (Fig. 16A, B) et un Doppler pulsé obtenu par échographie trans-thoracique (Fig. 16C, D), pour montrer la faisabilité de la procédure. Cela ne permet pas un monitorage continu mais permet de faire une photographie à un instant pour refléter l'état cardiovasculaire et le couplage ventriculo-artériel des patients.

*4.3 -Construction de la boucle avec une mesure non invasive de la pression.*

Il est également possible de construire des boucles Pre / Vél avec des moniteurs non-invasifs de la pression artérielle ne nécessitant pas la pose d'un cathéter intraartériel mais construisant la courbe de pression artérielle à l'aide d'un Tonomètre d'aplanation posé sur l'artère radiale, digitale ou brachiale. A titre d'exemples de tels capteurs, on peut citer le capteur digital et radial CNAP, le capteur digital *Clearsight Edwards* et le capteur radial et brachial *Shygmocor.*
L'utilisation de ce type de capteur, couplé à un moniteur Doppler type échographie cardiaque ou Doppler sus sternal, permet de construire la boucle Pre / Vél de manière totalement non-invasive.
Cela peut être particulièrement intéressant en-dehors du bloc opératoire ou de la réanimation, dans des services de cardiologie voire des cabinets de cardiologie de ville, pour estimer le couplage ventriculo-artériel des patients et suivre l'évolution selon les traitements utilisés.

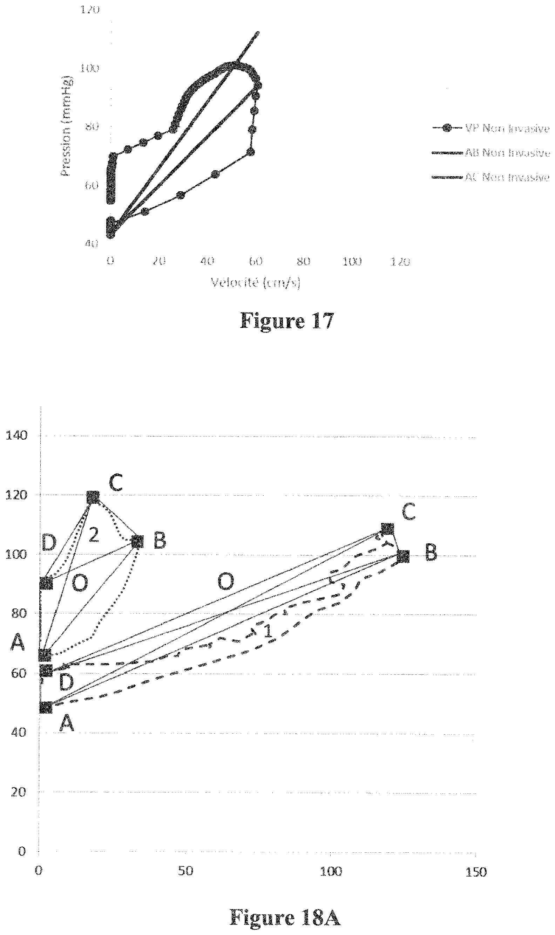

La figure 17 présente un exemple de boucle Pre / Vél construite avec un échographe cardiaque transthoracique et un capteur de pression *Digital Clearsight.*

**Exemple 5 : représentation graphique simplifiée de la boucle, sous forme d'un quadrilatère**

Dans ce qui précède, la boucle P/D, identique à un facteur constant près à la boucle Pre / Vél, a été représentée en discrimant quatre points importants, en formant deux droites et en mesurant trois angles.
Une autre représentation graphique est proposée ici, à l'aide des 4 points A, B, C, et D qui forment un quadrilatère convexe de centre O.
Par analogie à l'autre description, plus l'angle (COB ou AOD) entre les diagonales du quadrilatère est ouvert (>15°), plus le couplage du patient est mauvais et inversement. L'angle COB / AOD peut être assimilé à un angle de découplage car le couplage est d'autant moins bon que cet angle est plus grand.
Cette méthode visuelle avec juste la localisation des 4 points permet une estimation rapide du couplage. De plus elle ne nécessite pas la construction complète de la boucle mais juste le repérage des 4 points distinctifs A, B, C, D.
La figure 18A montre la différence de forme de quadrilatère entre un patient présentant un bon couplage (healthy, boucle 1) et un patient présentant un mauvait couplage ventriculo-artériel (comorbid, boucle 2).

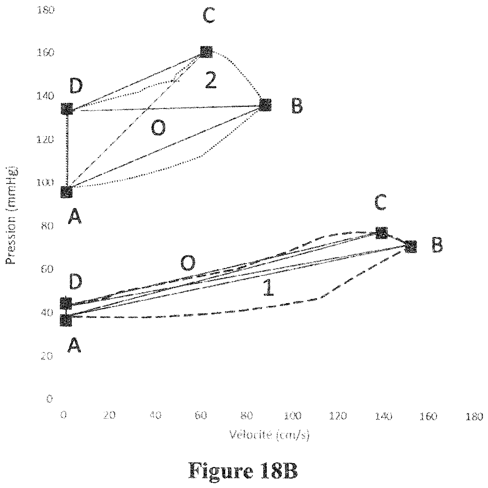

La figure 18B illustre le suivi d'un patient initialement en état d'hypotension avec vélocités accélérées (boucle 1) que l'on peut définir comme un état de vasodilatation excessive, et qui, après administration de vasoconstricteurs, se trouve dans un état d'hypertension avec vélocités réduites (boucle 2) que l'on peut définir comme un état de vasoconstriction excessive (le médicament utilisé n'était donc pas approprié). La surface du quadrilatère 2 (exprimé en mmHg $\times$ cm/s ou en $W/cm^2$ par application du coefficient $1,33 \times 10^4$) est bien plus grande que celle du quadrilatère 1, ainsi que l'angle AOD entre les diagonales, dit de découplage : 57° contre 3°. Bien entendu, l'angle $\gamma$ (angle BAC) est également être mesuré comme décrit précédemment.

**Tableau 7** : Paramètres des situations illustrées à la figure 18

|  | Surface | Angle de découplage | Angle gamma |
|---|---|---|---|
| Healthy | 1370 | 6° | 2,5° |
| Comorbid | 751 | 63° | 23° |
| quadrilatère 1 (vasoconstriction excessive) | 1068 | 57° | 19° |
| quadrilatère 2 (vasodilatation excessive) | 477 | 3° | 1,5° |

### Exemple 6 : Visualisation de la boucle en 3D

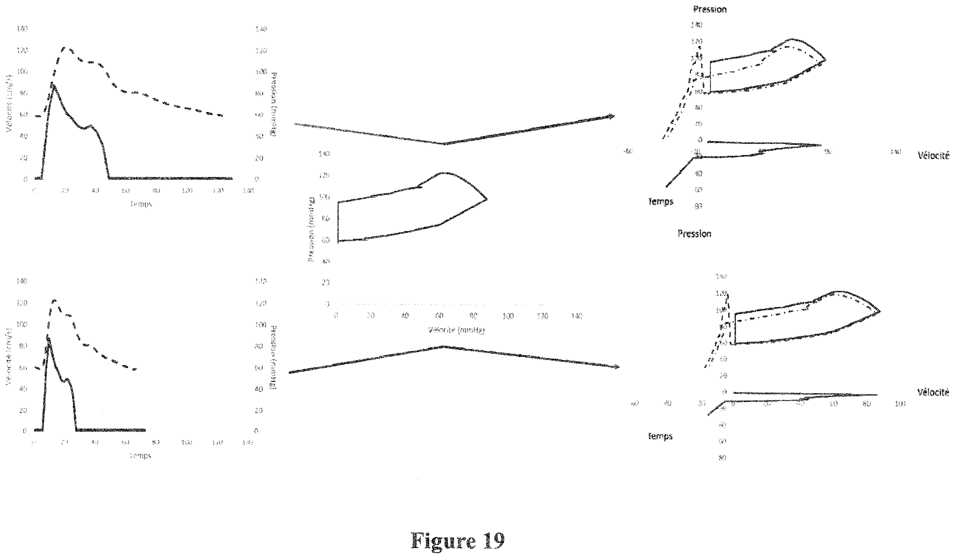

Il est ici proposé de visualiser la boucle Pre / Vél en 3D en intégrant le facteur temps dans un axe additionnel (allant vers l'opérateur dans la représentation présentée en Figure 19). Cela permet de prendre en compte la durée de systole. En effet, deux systoles différentes par leur durée, toutes caractéristiques de pression et de vélocité étant égales par ailleurs, présentent une relation Pression - Vélocité identique, représentée par une boucle identique en tout point. L'intégration du temps dans un graphique 3D permet de différencier ces deux situations, en tenant compte de la durée de la systole.

Une application de cette représentation pourrait être de visualiser la puissance effective par unité de surface aortique générée lors d'une systole. En effet, Le produit d'une pression par un débit représente une puissance. La vélocité correspondant à un débit par unité de surface, le produit d'une pression par une vélocité est une puissance par unité de surface. En intégrant chaque point de puissance calculé par unité de temps (3eme axe), on obtient le travail généré par une systole par unité de surface aortique.

### Exemple 7 : Diagramme de puissance et estimation du couplage ventriculo-aortique

#### 7.1 - Principes du diagramme de puissance

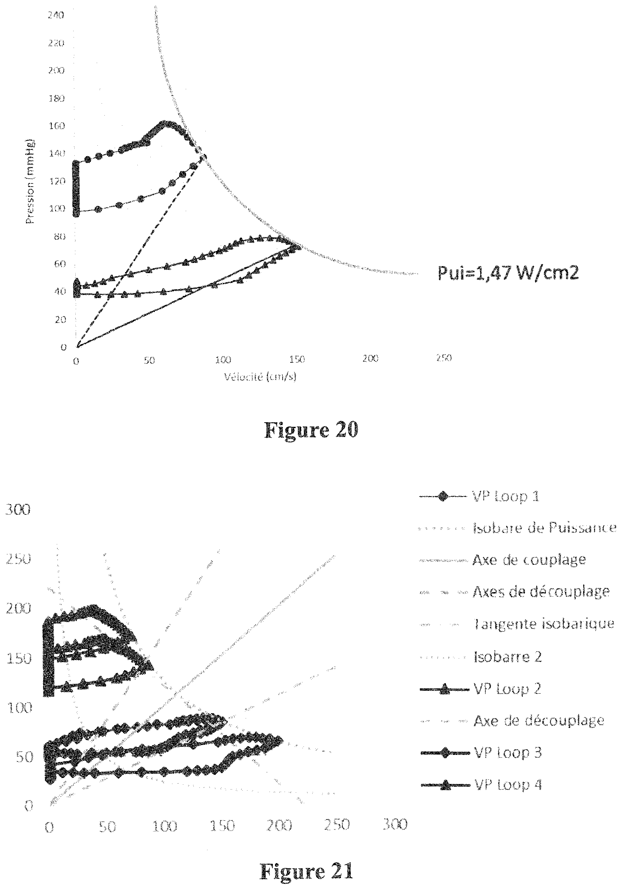

En hémodynamique, puisque la puissance est définie par le produit d'une pression et d'un débit, et que ce même débit est fonction de la surface aortique, alors le produit d'une vélocité par une pression peut représenter la puissance (Pui) produite par le système cardio-vasculaire par unité de surface aortique et être exprimé en $Watt/cm^2$. Sur le diagramme de la boucle Pre / Vél, on peut donc tracer des courbes Iso-Puissance liant les points où le produit Vel x Pré est identique. On peut alors facilement visualiser la puissance maximale générée par le patient (Figure 20). On peut voir que les deux boucles (• et A) génèrent la même puissance maximale ($Pui_{max}$) en ayant des profils très différents : la courbe • génère de la puissance avec essentiellement de la pression dans le circuit cardiovasculaire contrairement à la courbe ▲ qui génère essentiellement de la puissance avec une vélocité sanguine élevée.

Un bon couplage est obtenu lorsque la puissance maximale est fournie à parts égales par la vélocité et la pression, ce qui se traduit par une courbe passant par le sommet de l'hyperbole de puissance maximale. Une $Pui_{max}$ élevée au plus près du sommet de l'hyperbole pourrait traduire une bonne efficience du système cardio-vasculaire entre la vélocité du sang et la pression artérielle. On définit ainsi un « axe de couplage » passant par les sommets des courbes iso-puissance (Figure 21).

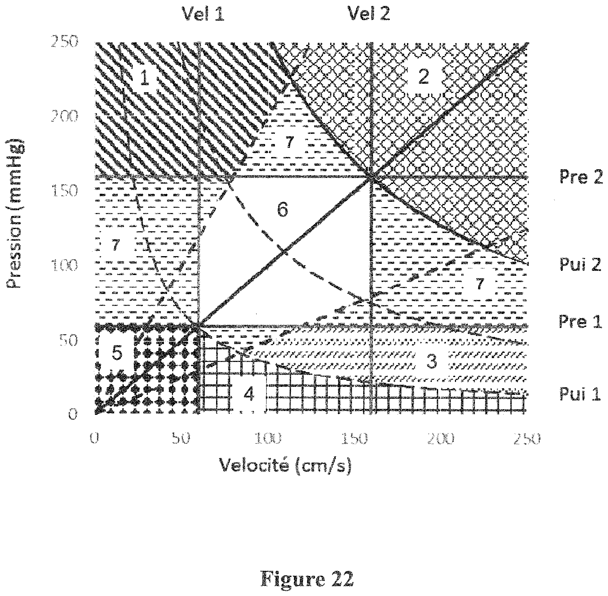

Le schéma présenté aux figures 21 et 22 permet d'avoir une vue d'ensemble de la puissance et de l'efficience éjectionelle produite par le système cardio-vasculaire et représentée par la boucle Pre / Vél. Il permet d'identifier un patients insuffisant cardiaque, donc produisant peu de $Pui_{max}$ et pouvant apparaitre faussement couplé sur l'analyse unique de la boucle Pre / Vél du fait d'une insuffisance de contractilité pour produire de la puissance.

Les axes de découplage peuvent servir comme limites indicatives de zones à risques de complication. Ceux-ci sont proportionnels aux paramètres pressions et vélocités, et les valeurs seuils qu'ils délimitent prennent en compte le potentiel de puissance maximal du système à cet instant donné. A titre d'exemple, deux axes de découplage ont été tracés sur la figure 22, correspondant à Pression = 2*Vélocité et Pression = ½*Vélocité.

Le schéma présenté à la figure 22 montre une représentation Pression/Vélocité, avec des zones à risques déduites de l'analyse du diagramme de puissances. Pour chaque zone, une préconisation thérapeutique peut être établie, comme décrit ci-dessous :

**Zone 1** : Situations à risque d'HTA maligne. La pression est élevée, aux dépens d'un débit inadapté, relativement bas. La puissance du système est physiologique, l'efficience du système mauvaise. Une thérapeutique par vasodilatateur pourrait améliorer la vélocité et donc le débit cardiaque, tout en diminuant la pression et en modifiant peu la puissance développée, améliorant ainsi l'efficience. Les médicaments préconisés dans ces conditions sont :

- Antihypertenseurs vasodilatateurs : Inhibiteurs calciques, Inhibiteurs de l'enzyme de conversion, Sartans, Dérivés nitrés, Alpha bloquants
- Diurétiques : Diurétiques de l'anse, Diurétique Thiazidiques, Inhibiteurs du récepteur au minéralocorticoïde ;
- Bêta-bloquants : Cardiosélectif ou non cardiosélectif.

Cette hypertension peut également être la conséquence d'une angoisse ou d'une douleur. Cela peut se rencontrer en anesthésie ou au cours d'un état de stress. Un traitement analgésique, anxiolitique et/ou un approfondissement de l'anesthésie peut améliorer l'efficience du système cardiovasculaire.

**Zone 2 :** Zone « supra-physiologique » - « Hyperhémique ». La puissance générée résulte en une vélocité aortique et une pression artérielle supérieure aux objectifs habituels. Le risque est à la rupture capillaire en cas de fragilité du réseau vasculaire d'aval. Dans ce cas, la priorité est à la baisse de la pression afin de protéger le cerveau. L'association de traitement antihypertenseurs et d'un beta-bloquant cardiosélectif pourrait permettre de faire diminuer la pression artérielle tout en prévenant une augmentation de vélocité trop importante. Lors des anesthésies générales, cette situation peut se rencontrer lors d'une hypnose ou d'une analgésie insuffisante, un approfondissement de l'anesthésie est nécessaire soit par des hypnotiques (Propofol, Halogénés ...) soit par des morphinomimétiques.

**Zone 3 :** Zone à risque d'hypoperfusion. En cas de vasodilatation excessive, la vélocité est élevée, la pression est basse, la puissance est normale ou haute. Le risque est à l'insuffisance de perfusion car la pression est insuffisante pour permettre de distribuer le sang aux organes de haute résistance. Le traitement de choix est le vasoconstricteur :

- Catecholamine alpha 1 agoniste
- Analogues de la somatostatine
- Vasopressine

**Zone 4** : Zone d'insuffisance circulatoire, la pression est basse, la vélocité est également diminuée mais dans une moindre mesure que dans la zone 5. La puissance générée est basse. Il existe une condition pathologique menaçante à court terme. Cette condition est liée soit à une hypovolémie importante, soit à une insuffisance cardiaque décompensée. Dans le premier cas, une expansion volémique suffirait à faire augmenter les deux paramètres et donc la puissance ; dans l'autre cas, une action inotrope positive permettrait de restaurer la pression et la vélocité grâce à une augmentation de puissance

**Zone 5 :** Zone d'insuffisance circulatoire sévère, la vélocité et la pression sont efondrées et la puissance également. Le patient est en défaillance circulatoire. Cette état peut être causé soit par une hypovolémie importante, soit par une insuffisance cardiaque décompensée. Dans le premier cas, une expansion volémique associée à des vasoconstricteurs pourrait faire augmenter les deux paramètres et donc la puissance ; dans l'autre cas, une action inotrope positive permettrait de restaurer la pression et la vélocité grâce à une augmentation de puissance

- Inotropes Positifs :

    - Catecholamine Alpha et Beta agonistes
    - Inhibiteurs des phosphodiestérases
    - Sensibilisateurs du calcium

- Expansion volémique :

- Colloïdes, Cristalloïdes, Gélatines
- Transfusion

- Vasoconstricteurs :

  - Catecholamine alpha 1 agoniste type noradrénaline

☐ **Zone 6** : Zone « Physiologique ». La puissance par unité de surface est intermédiaire et suffisante pour permettre des conditions circulatoires adéquates aux besoins de l'organisme. La répartition entre pression et vélocité aortique est grossièrement équivalente, et il n'y a pas ou peu de perte d'énergie.

▓ **Zones 7 :** Situations intermédiaires. La puissance du système est faible, ou forte, dépendant de la vélocité. La pression est dans les normes. Les conditions de circulation sont adéquates mais non optimales. Une action thérapeutique visant à moduler la vélocité permettrait d'améliorer l'efficience du système.

(i) Vélocité basse : Dans ce cas les traitements préconisés sont :

- Soluté de remplissage : Cristalloïdes, Colloïdes, Gélatines
- Inotropes : Catécholamines Beta 1 agoniste, inhibiteurs des phosphodiestérases, Sensibilisateurs du calcium

(ii) Vélocité élevée : Souvent, cette situation se présente au cours d'une anesthésie générale, ou lors de réanimations et fait état d'une situation hémodynamique complexe, ou bien lors des insuffisances cardiaques à haut débit compensées. Dans ce dernier cas, un traitement inotrope négatif semble être la molécule de choix comme un bêtabloquant.

### 7.2 - Exemples de patients et de suivi de traitement

Dans ces exemples, trois courbes iso-puissance sont figurées, représentant schématiquement des conditions limites des normes physiologiques généralement acceptées par les sociétés savantes. En effet, bien qu'aucune valeur seuil de l'hypotension artérielle ne soit consensuelle, il est généralement accepté qu'une PA systolique inférieure à 90 mmHg correspond à une hypotension. Le seuil d'hypertension est quant à lui défini par les sociétés de cardiologie comme une PA systolique > 140 mmHg. Concernant les vélocités, aucune valeur ne peut servir de référence formelle, mais il est communément accepté qu'une vélocité maximale systolique inférieure à 50 cm/s est inférieure à la normale, de même qu'une vélocité maximale systolique supérieure à 150 cm/s est supra-physiologique.
Les isobares de puissance par unité de surface limites sont donc construites en prenant en compte ces considérations, et ne représentent pas une valeur limite absolue, mais plutôt une limite indicative au-delà ou en-deçà desquelles des complications peuvent survenir.

- Ainsi, une courbe iso-puissance minimale équivalente à 0,5 W/cm$^2$, et une courbe iso-puissance maximale équivalente à 3,4 W/cm$^2$ sont figurées sur les prochains exemples. Une courbe iso-puissance de référence fixée à 1,6 W/cm$^2$ semble représenter une condition idéale, puisqu'elle correspond au produit d'une pression systolique normale (120 mmHg) multipliée par une vélocité normale (100 cm/s).
- Par ailleurs, l'axe de couplage représente la droite d'équation Pression = 1*Vélocité, et représente le sommet de l'hyperbole d'équation : Pression = Pui$_{max}$/(1*Velocité). En effet, les inventeurs ont observé que l'ordre de grandeur de la vélocité aortique en cm/s était équivalent à l'ordre de grandeur de pression artérielle en mmHg.
- Deux axes de découplage ont été définis. A titre d'exemple non limitatif, les inventeurs ont défini un découplage Pression-Vélocité quand Pression = 2*Vélocité, ou quand Pression = ½*Vélocité

### 7.2.1 : Vasodilatation

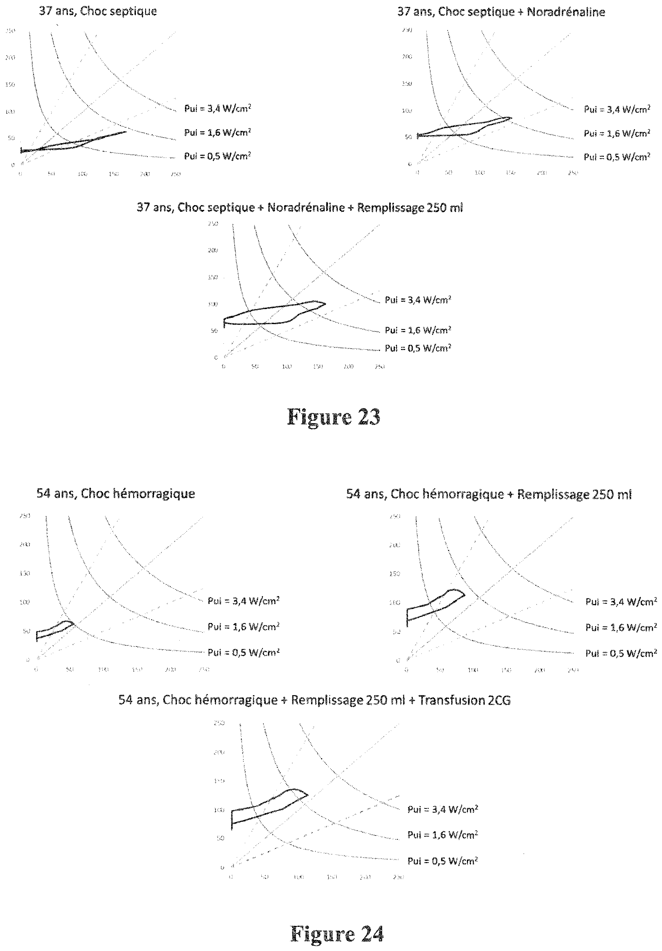

Il s'agit d'un patient de 37 ans, en choc septique, hypotendu après la réanimation initiale. PA = 65/35 mmHg, en hyperdébit avec une vélocité maximale = 160 cm/s. La puissance générée par le système est normale correspondant à 1,54 W/cm$^2$. En revanche la pression artérielle est insuffisante pour assurer la perfusion des différents organes. Après titration des vasoconstricteurs (administration répétée de vasoconstricteurs jusqu'à obtention de l'effet escompté), l'objectif de pression a été obtenu (pression artérielle moyenne = 65 mmHg), et associé à une réduction faible de la vélocité maximale (152 cm/s). La puissance générée est supérieure à la condition précédente (1,65 W/cm$^2$), et permet de

satisfaire l'objectif de pression de perfusion des organes. Après nouvelle épreuve de remplissage, la vélocité maximale augmente jusqu'à la valeur initiale de 160 cm/s, permettant d'obtenir une puissance par unité de surface de 2 W/cm$^2$. Graphiquement, comme illustré sur la figure 23, le résultat de la réanimation hémodynamique du patient est directement observable, avec une valeur de puissance proche de valeurs que l'on pourrait qualifier de physiologiques, et satisfaisant des objectifs de pression et de débit physiologiques (entre les limites de découplage).

### 7.2.2 : Hypovolémie

Dans cet exemple, illustré figure 24, il s'agit d'un patient de 54 ans souffrant d'un choc hémorragique, la pression et la vélocité initiales sont basses, associées à une faible puissance. Le traitement initial consiste en une expansion volémique, permettant d'améliorer significativement la pression et la vélocité, faisant changer le système du patient de puissance. Une transfusion supplémentaire permet d'améliorer encore son hémodynamique.

### 7.2.3 : défaut Contractilité

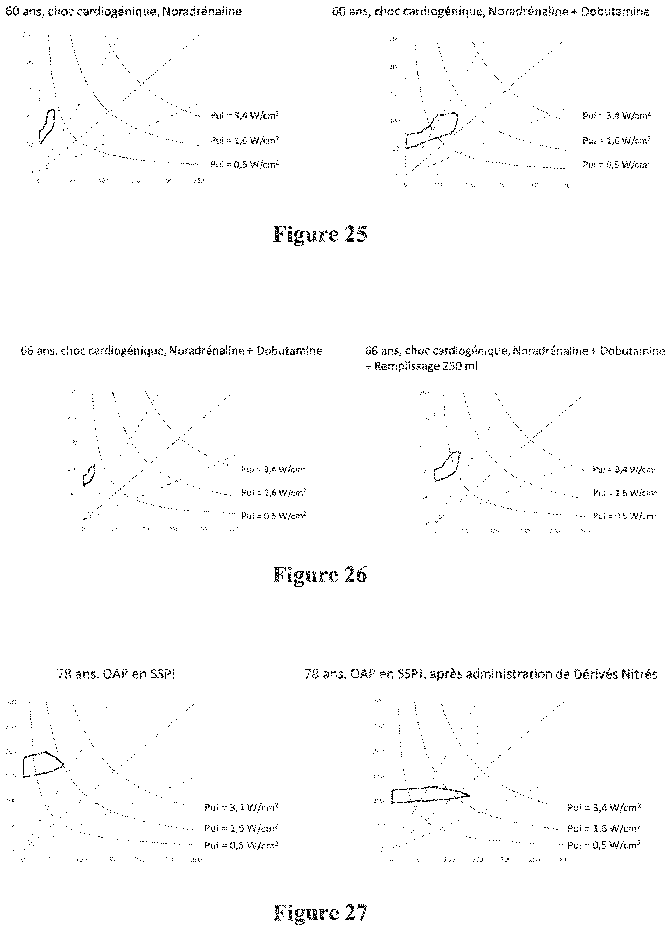

Dans l'exemple illustré en figure 25, il s'agit d'un patient de 60 ans hospitalisé pour décompensation d'une insuffisance cardiaque. La stratégie initiale avait consisté en une expansion volémique et l'introduction de noradrénaline car le patient était hypotendu. Ainsi, la pression artérielle est restaurée dans les limites de la normalité, mais la vélocité et donc le débit cardiaque restent insuffisants pour permettre des conditions circulatoires efficaces. La puissance est faible. L'ajout d'un inotrope permet de restaurer efficacement l'hémodynamique du patient, en augmentant significativement la vélocité maximale, pour la même gamme de pression artérielle.

Dans l'exemple illustré en figure 26, il s'agit d'un patient de 66 ans en postopératoire d'une chirurgie cardiaque hémorragique, présentant une insuffisance cardiaque nécessitant l'introduction d'inotrope en fin d'intervention. A l'arrivée en réanimation, le patient est instable, avec une vélocité et une puissance basse. Après remplissage, le patient présente une amélioration de ses conditions circulatoires, avec une augmentation significative de la pression, de la vélocité et donc de la puissance. Il est probable qu'une diminution de Noradrénaline, ou la poursuite du remplissage, pourrait permettre d'améliorer encore l'état du patient.

### 7.2.4 : Vasoconstriction

Dans cet exemple, illustré en figure 27, il s'agit d'une patiente de 78 ans, hospitalisée en chirurgie orthopédique pour fracture du col fémoral. La patiente présente des antécédents d'oedème aigu du poumon. En post opératoire immédiat, alors que la patiente a nécessité une transfusion pendant l'intervention, elle présente une dégradation de son état respiratoire associée à une augmentation progressve de la pression artérielle. Parallèlement, la vélocité avait diminué (la patiente étant toujours sous anesthésie générale car instable sur le plan respiratoire), tout en restant dans les limites inférieures à la normale (60 cm/s). La conséquence est une puissance normale. Après administration de vasodilatateur, nous avons observé une diminution de la pression artérielle, et une augmentation significative de la vélocité, une augmentation de puissance, le point au pic de vélocité se rapprochant de l'axe de couplage, les conditions circulatoires se sont améliorées, confirmée par une amélioration de l'état respiratoire, permettant un réveil complet de la patiente quelques minutes après le traitement.

## Exemple 8 : Fonction de transfert pour le calcul des paramètres de la

## boucle Pré / Vel

### 8.1 - Importance d'utiliser une fonction de transfert pour le calcul des paramètres de la boucle Pré / Vel

La boucle Pré / Vel peut être construite avec une mesure Doppler dans l'aorte thoracique descendante (Doppler oesophagien) ou une mesure Doppler dans l'aorte thoracique ascendante (Doppler sus sternal ou échographie cardiaque transthoracique ou transoesophagienne). La mesure de la pression peut se faire en radial, brachial ou fémoral. Les trois exemples ci-dessous montrent que les paramètres de la boucle (angles et surfaces) peuvent changer lorsque la pression est mesurée au niveau du Doppler en central et non pas en périphérie. Ceci est dû au phénomène d'amplification de la pression de la centrale vers la périphérie due à la rigidité aortique et aux ondes de réflexion.

Les trois exemples de mesures concomitantes de la pression en radial et en aortique couplée à la mesure du Doppler en aortique illustrent que suivant les cas, les paramètres de la boucle peuvent être similaires avec une artère périphérique par rapport à la centrale, mais peuvent également différer de façon significative.

Exemples de boucle Pré / Vel avec pression mesurée en centrale (•) et pression mesurée en périphérie (▲) .

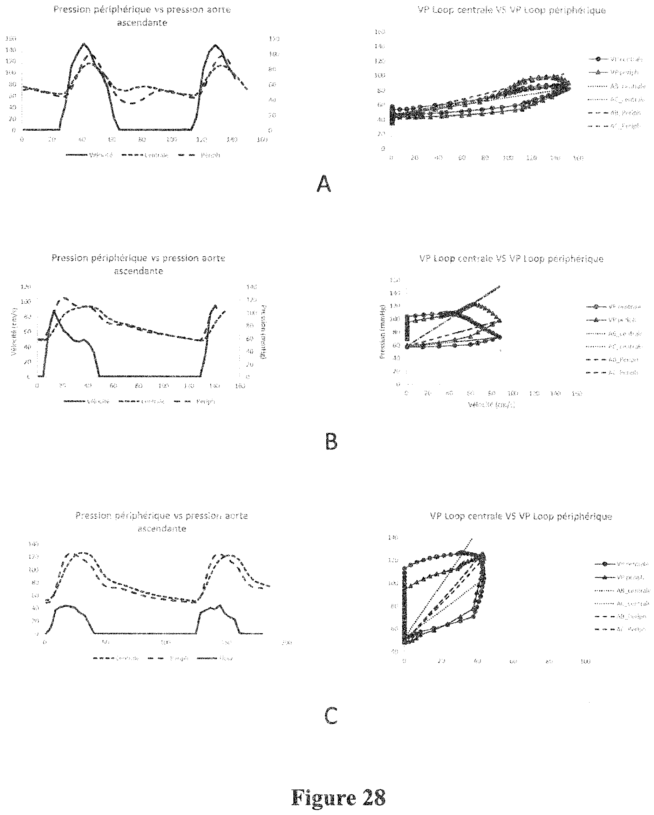

Exemple 1 (figure 28A) : Patient jeune 21 ans : peu de changement des paramètres de la Loop vel/pre centrale vs périphérie

Exemple 2 (figure 28B) : Patient de 45 ans : amplification précoce de la pression en périphérie augmentant l'angle alpha et diminuant l'angle gamma.

Exemple 3 (figure 28C) : Patient de 65 ans avec risque cardiovasculaire : amplification ultra précoce de la pression en périphérie faisant coïncider le pic de pression maximale avec le pic de vélocité maximale, alors que chez ce type de patient le pic central de pression systolique est physiologiquement très retardé par rapport à celui du flux. Cela donne un effet faussement couplé sur la boucle ▲ alors qu'en réalité la boucle est de forme carrée avec un angle Gamma important.

Ces trois exemples démontrent l'importance de construire les boucles Pré / Vel en mesurant la vélocité du sang et la pression artérielle au même endroit. En pratique clinique quotidienne, la vélocité est mesurée en centrale au niveau de l'Aorte thoracique soit ascendante soit descendante, et la pression est mesurée en périphérie soit fémorale soit radiale. Il est donc nécessaire d'utiliser des fonctions de transfert mathématique permettant de recréer la courbe de pression centrale à partir d'une courbe de pression périphérique mesurée de manière invasive ou non.

Quatre fonctions de transfert sont donc nécessaires en fonction de l'endroit de mesure du flux et de la pression. Deux fonctions pour reconstruire la pression radiale soit pour l'aorte thoracique ascendante soit descendante et deux fonctions pour reconstruire la pression fémorale soit pour l'aorte thoracique ascendante soit descendante.

### 8.2- Elaboration des fonctions de transfert

*Méthodologie:*

Des signaux de pression artérielle ont été recueillis au niveau des artères radiale et fémorale (dénommées « périphériques ») et au niveau de l'aorte ascendante et descendante (dénommées « centrales »). Le but de ce travail était d'évaluer des fonctions de transfert permettant de reconstruire les formes d'ondes centrales à partir d'un signal enregistré dans les artères périphériques chez des patients sous anesthésie générale dans différentes conditions hémodynamiques.

Les données ont été recueillies de manière synchronisée, échantillonées à 125 Hz. Ensuite, les signaux ont été découpés battement par battement en prenant comme début de cycle le maximum local de la seconde dérivée, correspondant à l'accélération maximale de l'onde de pression.

Les ondes périphériques et centrales ont ensuite servi à estimer les fonctions de transfert en adaptant un modèle autorégressif (ARX = *autoregressive exogenous*) [5]. Ce modèle consiste à évaluer les paramètres $a_{na}$ et $b_{nb}$ par la méthode des moindres carrés afin de résoudre l'équation suivante :

$$y(t)+a_1 y(t-1)+...+a_{na} y(t-na) = b_1 u(t)+...+b_{nb} u(t-nb+1)+e(t)$$

« na » et « nb » sont le nombre de paramètres a et b. Les paramètres b correspondent au numérateur et les paramètres a correspondent au dénominateur de la fonction de transfert. Ils représentent donc respectivement les zéros et les pôles du système. Après plusieurs essais, nous les inventeurs ont obtenu les meilleurs résultats en fixant na = nb = 5.

La formule s'écrit alors comme suit :

$$y(t)+a_1 y(t-1)+ a_2 y(t-2)+ a_3 y(t-3)+ a_4 y(t-4)+ a_5 y(t-5) = b_1 u(t)+ b_2 u(t-1) +b_3 u(t-2)+ b_4 u(t-3) +b_5 u(t-4)$$

u(t) est le signal d'entrée en fonction du temps : la pression périphérique.
y(t) est le signal de sortie en fonction du temps : la pression centrale recherchée.

Les inventeurs ont estimé des fonctions de transfert à partir de toutes pressions mesurées puis estimé la concordance avec un *normalized root mean square error* (NRMSE) qui va de moins l'infini (concordance très mauvaise) à 100 % (concordance parfaite). Les fonctions de transfert qui présentent le meilleur NRMSE en médiane ont été conservées.

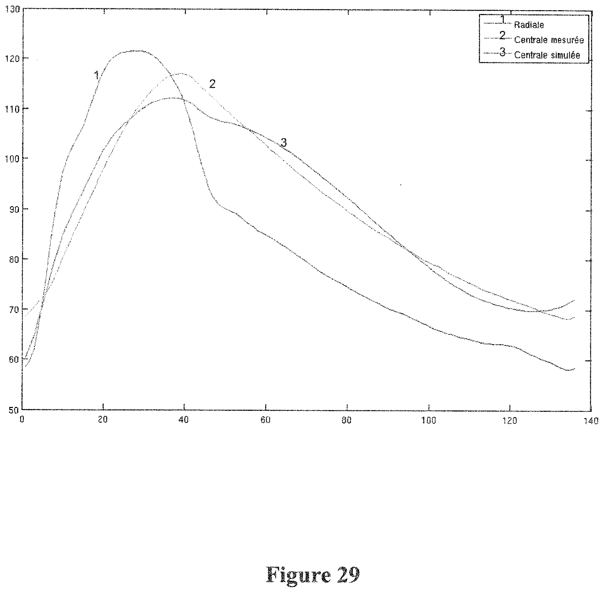

Les inventeurs ont analysé les données recueillis chez 5 patients pour créer les 4 fonctions de transfert et ont confirmé les résultats chez 20 autres patients (Figure 29).

De plus, le modèle a également été validé sur 4 patients pour qui la pression périphérique était mesurée par un capteur non-invasif de pression type Clearsight, Edwards©.

*Fonctions de Transfert:*

Artère fémorale - Aorte ascendante :

Paramètres « b » : 0.3771, -0.5432, -0.0140, 0.2922, -0.0921
Paramètres « a » : -2.0934, 1.1795, 0.4089, -0.6981, 0.2232

Artère fémorale - Aorte descendante

Paramètres « b » : 0.1467, -0.0291, -0.2380, 0.1118, 0.0297
Paramètres « a » : -2.1340, 1.2873, 0.3845, -0.7939, 0.2772

Artère radiale - Aorte ascendante

Paramètres « b » : 0.2433, -0.4522, 0.3060, -0.0431, -0.0228
Paramètres « a » : -1.7255, 0.7454, 0.4620, -0.6547, 0.2009

Artère radiale - Aorte descendante

Paramètres « b » : 0.3778, -0.5649, 0.1729, 0.0950, -0.0480
Paramètres « a » : -2.0489, 1.4535, -0.0771, -0.6101, 0.3127

**REFERENCES**

1- High versus low blood-pressure target in patients with septic shock. Asfar P, Meziani F, Hamel JF, Grelon F, Megarbane B, Anguel N, Mira JP, Dequin PF, Gergaud S, Weiss N, Legay F, Le Tulzo Y, Conrad M, Robert R, Gonzalez F, Guitton C, Tamion F, Tonnelier JM, Guezennec P, Van Der Linden T, Vieillard-Baron A, Mariotte E, Pradel G, Lesieur O, Ricard JD, Hervé F, du Cheyron D, Guerin C, Mercat A, Teboul JL, Radermacher P; SEPSISPAM Investigators. N Engl J Med. 2014 Apr 24;370(17):1583-93.
2- Improving perioperative outcomes: fluid optimization with the esophageal Doppler monitor, a metaanalysis and review. Phan TD, Ismail H, Heriot AG, Ho KM. J Am Coll Surg. 2008 Dec; 207(6):935-41.
3- Assessment of systolic and diastolic ventricular properties via pressure-volume analysis: a guide for clinical, translational, and basic researchers. Daniel Burkhoff, Israel Mirsky and Hiroyuki Suga. Am J Physiol Heart Cire Physiol, 289:H501-H512, 2005.
4- Real-time Doppler-based arterial vascular impédance and peripheral pressure-flow loops: a pilot study. Thiele RH, Bartels K, Esper S, Ikeda K, Gan TJ. J Cardiothorac Vasc Anesth. 2014 Feb;28(1):36-41.
5- Ljung L. System Identification: Theory for the User. 2 édition. Upper Saddle River, NJ: Prentice Hall; 1999. 672 p.
6- Efficiency of energy transfer from pressure-volume area to external mechanical work increases with contractile state and decreases with afterload in the left ventricle of the anesthetized closed-chest dog. Nozawa T, Yasumura Y, Futaki S, Tanaka N, Uenishi M, Suga H, Circulation 1988 May; 77(5): 1116-1124.

**Revendications**

1. Dispositif pour évaluer le couplage ventriculo-artériel d'un patient en temps réel, **caractérisé en ce qu'**il comprend des moyens informatiques paramétrés pour :

(i) recevoir et traiter, en temps réel, des signaux provenant, d'une part, de moyens de mesure du flux sanguin artériel et d'autre part, de moyens de mesure de la pression artérielle ;
(ii) afficher, en temps réel, le tracé d'au moins une boucle pression / flux artériel ;
(iii) déterminer, sur la dernière boucle pression / flux complétée, les coordonnées des points suivants :

A : point de la boucle où le flux est nul et la pression minimale,
B : point de la boucle où le flux est le plus important,

C : point de la boucle où la pression est la plus grande,

D : point de la boucle où le flux est nul et la pression maximale

(iv) calculer le pourcentage de l'aire de la boucle (Stot) occupé par la partie S2 située entre les droites (AB) et (AC) et/ou l'angle $\gamma$ entre ces deux droites et/ou l'angle aigu $\delta$ entre les droites (AC) et (BD) ;

(v) interpréter les résultats de la façon suivante :

si S2/Stot $\leq$ R2b et/ou $\gamma \leq$ Gb et/ou $\delta \leq$ Db, le patient présente un bon couplage ventricule artériel ;

si S2/Stot $\geq$ R2m et/ou $\gamma \geq$ Gm et/ou $\delta \geq$ Dm, le patient présente un mauvais couplage ventricule artériel ;

dans une situation intermédiaire, la qualité du couplage ventricule artériel du patient est intermédiaire,

R2b, R2m, Gb, Gm, Db et Dm étant des seuils prédéterminés.

2. Dispositif selon la revendication 1, dans lequel les moyens de mesure du flux sanguin artériel comprennent un appareil Doppler oesophagien ou un appareil Doppler sus-sternal ou des moyens d'échographie cardiaque transthoracique ou transoesophagienne.

3. Dispositif selon la revendication 1 ou la revendication 2, dans lequel les moyens de mesure de la pression artérielle comprennent un cathéter de mesure de la pression artériel radial ou fémoral, ou un tonomètre non invasif.

4. Dispositif selon l'une quelconque des revendications 1 à 3, **caractérisé en ce que** les moyens informatiques sont également paramétrés pour calculer la pression centrale aortique, à partir de la pression artérielle mesurée.

5. Dispositif selon l'une quelconque des revendications 1 à 4, **caractérisé en ce que** les moyens informatiques sont également paramétrés pour afficher l'évolution de S2 et/ou de $\gamma$ et/ou de $\delta$ en fonction du temps.

6. Dispositif selon l'une quelconque des revendications 1 à 5, **caractérisé en ce que** les moyens informatiques sont paramétrés pour afficher des courbes iso-puissance.

7. Dispositif selon l'une quelconque des revendications 1 à 6, **caractérisé en ce que** les moyens informatiques sont paramétrés pour afficher le tracé d'une boucle pression / flux artériel en fonction du temps, le temps étant représenté par un troisième axe.

## Patentansprüche

1. Vorrichtung zur Beurteilung der ventrikuloarteriellen Kopplung eines Patienten in Echtzeit, **dadurch gekennzeichnet, dass** sie Informatikmittel umfasst, die so parametrisiert sind, dass sie:

(i) in Echtzeit Signale, die einerseits von Mitteln zur Messung des arteriellen Blutflusses und andererseits von Mitteln zur Messung des arteriellen Drucks stammen, empfangen und verarbeiten;

(ii) den Verlauf mindestens einer arteriellen Druck-/Fluss-Schleife in Echtzeit anzeigen;

(iii) an der letzten abgeschlossenen Druck-/Fluss-Schleife die Koordinaten der folgenden Punkte bestimmen:

A: Punkt der Schleife, an dem der Fluss null und der Druck minimal ist,

B: Punkt der Schleife, an dem der Fluss am größten ist,

C: Punkt der Schleife, an dem der Druck am höchsten ist,

D: Punkt der Schleife, an dem der Fluss null und der Druck maximal ist,

(iv) den Prozentanteil der Fläche der Schleife (Stot), der von dem zwischen den Geraden (AB) und (AC) liegenden Teil S2 eingenommen wird, und/oder den Winkel $\gamma$ zwischen diesen zwei Geraden und/oder den spitzen Winkel $\delta$ zwischen den Geraden (AC) und (BD) berechnen;

(v) die Ergebnisse wie folgt interpretieren:

wenn S2/Stot $\leq$ R2b und/oder $\gamma \leq$ Gb und/oder $\delta \leq$ Db, weist der Patient eine gute ventrikuloarterielle Kopplung auf;

wenn S2/Stot $>_-$ R2m und/oder $\gamma \geq$ Gm und/oder $\delta \geq$ Dm, weist der Patient eine schlechte ventrikuloarterielle Kopplung auf;

in einem dazwischenliegenden Fall ist die Qualität der ventrikuloarteriellen Kopplung des Patienten dazwischenliegend,

wobei R2b, R2m, Gb, Gm, Db und Dm vorbestimmte Schwellenwerte sind.

2. Vorrichtung nach Anspruch 1, wobei die Mittel zur Messung des arteriellen Blutflusses eine Ösophagusdoppler-Einrichtung oder eine suprasternales Doppler-Einrichtung oder Mittel für transthorakale oder transösophageale Echokardiographie umfassen.

3. Vorrichtung nach Anspruch 1 oder Anspruch 2, wobei die Mittel zur Messung des arteriellen Blutdrucks einen Radialis- oder Femoraliskatheter zur Messung des arteriellen Drucks, oder ein nichtinvasives Tonometer umfassen.

4. Vorrichtung nach einem der Ansprüche 1 bis 3, **dadurch gekennzeichnet, dass** die Informatikmittel ebenfalls so parametrisiert sind, dass sie anhand des gemessenen arteriellen Drucks den zentralen Aortendruck berechnen.

5. Vorrichtung nach einem der Ansprüche 1 bis 4, **dadurch gekennzeichnet, dass** die Informatikmittel ebenfalls so parametrisiert sind, dass sie die Entwicklung von S2 und/oder $\gamma$ und/oder $\delta$ in Abhängigkeit von der Zeit anzeigen.

6. Vorrichtung nach einem der Ansprüche 1 bis 5, **dadurch gekennzeichnet, dass** die Informatikmittel so parametrisiert sind, dass sie Iso-Leistungskurven anzeigen.

7. Vorrichtung nach einem der Ansprüche 1 bis 6, **dadurch gekennzeichnet, dass** die Informatikmittel so parametrisiert sind, dass sie den Verlauf einer arteriellen Druck-/Fluss-Schleife in Abhängigkeit von der Zeit anzeigen, wobei die Zeit durch eine dritte Achse dargestellt wird.

## Claims

1. A device for evaluating the ventricular-arterial coupling of a patient in real time, **characterized in that** it comprises computer means parameterized for:

(i) receiving and treating, in real time, signals originating, on the one hand, from means for measuring arterial blood flow and, on the other hand, from means for measuring arterial pressure;
(ii) displaying, in real time, the plot of at least one arterial pressure/flow loop;
(iii) determining, on the final pressure/flow loop completed, the coordinates of the following points:

A: point of the loop where the flow is zero and the pressure is at the minimum,
B: point of the loop where the flow is highest,
C: point of the loop where the pressure is highest,
D: point of the loop where the flow is zero and the pressure is at the maximum;

(iv) calculating the percentage of the area of the loop (Stot) occupied by the part S2 located between the straight lines (AB) and (AC) and/or the angle $\gamma$ between these two straight lines and/or the acute angle $\delta$ between the straight lines (AC) and (BD);
(v) interpreting the results in the following way:

if S2/Stot $\leq$ R2b and/or $\gamma \leq$ Gb and/or $\delta \leq$ Db, the patient shows good ventricular-arterial coupling;
if S2/Stot $\geq$ R2m and/or $\gamma \geq$ Gm and/or $\delta \geq$ Dm, the patient shows poor ventricular-arterial coupling;
in an intermediate situation, the quality of the ventricular-arterial coupling of the patient is intermediate,

R2b, R2m, Gb, Gm, Db and Dm being predetermined thresholds.

2. The device as claimed in claim 1, wherein the means for measuring arterial blood flow comprise an esophageal Doppler device or a suprasternal Doppler device or means for transthoracic or transesophageal cardiac echography.

3. The device as claimed in claim 1 or claim 2, wherein the means for measuring arterial pressure comprise a catheter for measuring radial or femoral arterial pressure, or a noninvasive tonometer.

4. The device as claimed in any one of claims 1 to 3, **characterized in that** the computer means are also parameterized for calculating the aortic central pressure, from the arterial pressure measured.

5. The device as claimed in any one of claims 1 to 4, **characterized in that** the computer means are also parameterized for displaying the evolution of S2 and/or of $\gamma$ and/or of $\delta$ as a function of time.

6. The device as claimed in any one of claims 1 to 5, **characterized in that** the computer means are parameterized for displaying iso-power curves.

7. The device as claimed in any one of claims 1 to 6, **characterized in that** the computer means are parameterized for displaying the plot of an arterial pressure/flow loop as a function of time, the time being represented by a third axis.

Figure 1

Figure 2

**Figure 3**

**Figure 4**

**Figure 5**

**Figure 6**

Figure 7

Figure 8

Figure 9

Figure 10

Figure 11

Figure 12

Figure 13

Figure 14

Angle Alpha = 13°; Angle Beta = 16°; Angle Gamma = 3°

Angle Alpha = 28°; Angle Beta = 64°; Angle Gamma = 36°

Angle Alpha = 3°; Angle Beta = 4°; Angle Gamma = 1°

Angle Alpha = 5°; Angle Beta = 13°; Angle Gamma = 8°

**Figure 15**

**Figure 16**

**Figure 17**

**Figure 18A**

**Figure 18B**

Figure 19

Pui=1,47 W/cm2

**Figure 20**

——◆—— VP Loop 1

·······  Isobare de Puissance

———— Axe de couplage

— — — Axes de découplage

— · — Tangente isobarique

·······  Isobarre 2

——▲—— VP Loop 2

— — — Axe de découplage

——◆—— VP Loop 3

——▲—— VP Loop 4

**Figure 21**

Figure 22

38

37 ans, Choc septique

37 ans, Choc septique + Noradrénaline

37 ans, Choc septique + Noradrénaline + Remplissage 250 ml

## Figure 23

54 ans, Choc hémorragique

54 ans, Choc hémorragique + Remplissage 250 ml

54 ans, Choc hémorragique + Remplissage 250 ml + Transfusion 2CG

## Figure 24

60 ans, choc cardiogénique, Noradrénaline

60 ans, choc cardiogénique, Noradrénaline + Dobutamine

Pui = 3,4 W/cm²
Pui = 1,6 W/cm²
Pui = 0,5 W/cm²

Pui = 3,4 W/cm²
Pui = 1,6 W/cm²
Pui = 0,5 W/cm²

## Figure 25

66 ans, choc cardiogénique, Noradrénaline + Dobutamine

66 ans, choc cardiogénique, Noradrénaline + Dobutamine + Remplissage 250 ml

Pui = 3,4 W/cm²
Pui = 1,6 W/cm²
Pui = 0,5 W/cm²

Pui = 3,4 W/cm²
Pui = 1,6 W/cm²
Pui = 0,5 W/cm²

## Figure 26

78 ans, OAP en SSPI

78 ans, OAP en SSPI, après administration de Dérivés Nitrés

Pui = 3,4 W/cm²
Pui = 1,6 W/cm²
Pui = 0,5 W/cm²

Pui = 3,4 W/cm²
Pui = 1,6 W/cm²
Pui = 0,5 W/cm²

## Figure 27

A

B

Figure 28

41

Figure 29

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 03037181 A2
- US 20070161914 A1

**Littérature non-brevet citée dans la description**

- **ASFAR P ; MEZIANI F ; HAMEL JF ; GRELON F ; MEGARBANE B ; ANGUEL N ; MIRA JP ; DEQUIN PF ; GERGAUD S ; WEISS N.** High versus low blood-pressure target in patients with septic shock. *SEPSISPAM Investigators. N Engl J Med.,* 24 Avril 2014, vol. 370 (17), 1583-93
- **PHAN TD ; ISMAIL H ; HERIOT AG ; HO KM.** Improving perioperative outcomes: fluid optimization with the esophageal Doppler monitor, a metaanalysis and review. *J Am Coll Surg.,* Décembre 2008, vol. 207 (6), 935-41
- **DANIEL BURKHOFF ; ISRAEL MIRSKY ; HIROYUKI SUGA.** Assessment of systolic and diastolic ventricular properties via pressure-volume analysis: a guide for clinical, translational, and basic researchers. *Am J Physiol Heart Cire Physiol,* 2005, vol. 289, H501-H512
- **THIELE RH ; BARTELS K ; ESPER S ; IKEDA K ; GAN TJ.** Real-time Doppler-based arterial vascular impédance and peripheral pressure-flow loops: a pilot study. *J Cardiothorac Vasc Anesth.,* Février 2014, vol. 28 (1), 36-41
- **LJUNG L.** System Identification: Theory for the User. Prentice Hall, 1999, 672
- **NOZAWA T ; YASUMURA Y ; FUTAKI S ; TANAKA N ; UENISHI M ; SUGA H.** Efficiency of energy transfer from pressure-volume area to external mechanical work increases with contractile state and decreases with afterload in the left ventricle of the anesthetized closed-chest dog. *Circulation,* Mai 1988, vol. 77 (5), 1116-1124